# EUROPEAN PATENT SPECIFICATION

(11) **EP 1 948 236 B1**
(45) Date of publication and mention of the grant of the patent: **03.03.2021**
(21) Application number: 06849473.1
(22) Date of filing: 04.08.2006
(51) Int. Cl.: A61P 17/00, A61K 31/19, A61K 33/00, A61K 45/06, A61K 9/06, A61K 9/00

(54) **METHODS AND COMPOSITIONS FOR TREATMENT OF SKIN**
METHODEN UND ZUSAMMENSETZUNGEN ZUR BEHANDLUNG DER HAUT
PROCEDES ET COMPOSITIONS POUR LE TRAITEMENT DE LA PEAU

(30) Priority: 27.09.2005 WO PCT/IB2005/003665
(43) Date of publication of application: 30.07.2008
(73) Proprietor: Special Water Patents B.V., 3903 LW Veenendaal (NL)
(72) Inventor: DE RIJK, Jan, 3903 LW Veenendaal (NL)
(74) Representative: Huygens, Arthur Victor
(86) International application number: PCT/IB2006/004122
(87) International publication number: WO 2007/099398

(56) References cited:
- EP-A- 0 441 057
- EP-A1- 0 217 975
- EP-B1- 1 107 766
- DE-A1- 3 705 894
- GB-A- 1 367 359
- JP-B2- 3 227 378
- PATENT ABSTRACTS OF JAPAN vol. 2000, no. 05, 14 September 2000 (2000-09-14) & JP 2000 063894 A (DAISAN KOGYO KK; SHOWA DENKO KK), 29 February 2000 (2000-02-29)

## Description

### FIELD OF THE INVENTION

The present invention relates to compositions for the treatment of human skin, particularly facial skin, to alleviate the symptoms of cosmetic or dermatologic skin conditions and to improve the appearance and the feel of skin. The invention relates to compositions for treating and ameliorating skin conditions including acne, rosacea and wrinkling caused by photodamage, aging, hormonal imbalances, hyper-pigmentation, melasma, keratosis or the like.

### BACKGROUND OF THE INVENTION

There are a variety of skin conditions-whether cosmetic or as a result of disease-that cause individuals substantial discomfort or embarrassment, making effective treatment desirable. Among these conditions are acne, rosacea, aging, skin discoloration and photodamage, psoriasis, dandruff and eczema, and dermatitis.

Acne vulgaris is an inflammatory dermatological disorder that occurs frequently in adolescence and with some regularity in older adults. The condition can include skin lesions ranging from the comedo in a pilosepaceous follicle, to more severe symptoms such as pustules, papules, cysts and nodules. The condition can be uncomfortable and embarrassing, and can result in scarring and facial disfigurement. The pathology is believed to involve a number of factors. The first is formation of comedones (more commonly referred to as whiteheads) and blackheads, made up of solid horny masses that plug follicles and are associated with increased production of sebum. As keratinized cells continue to accumulate, pressure builds up within the follicles and eventually rupture. Horny material, sebum and bacteria are dumped into the skin, provoking inflammatory responses which take the form of pustules or cystic nodules.

There are many different forms of treatment for acne vulgaris. Simple washing and cleansing is one known method. It is known in the art to use topical anti-acne agents, such as salicylic acid and benzoyl peroxide can be used to treat acne. Further, retinoids and retinols may be used, but have the undesirable side effects of mild to severe irritation, redness, peeling and itching and burning. (See U.S. Patent No. 4,877,805 and U.S. Patent No. 4,355,028, for example).

Another skin condition requiring treatment is rosacea. Rosacea is a common chronic skin condition characterized by a spectrum of clinical indications including flushing episodes, erythema, telangiectasia, inflammatory papulopustular eruptions resembling acne, and ocular symptoms. See U.S. Patent No. 6,723,755, Chomczynski, et al. The etiology of rosacea is unknown, but it is presumed to be a genetically determined anomalous vascular response that develops in the third to sixth decades of life. The hypothesis that the basic pathogenesis of the disease is a flushing disorder is based on several findings. The disease appears to more prevalent in northern climates where cold exposure is experienced more often, and in light-skinned persons in whom flushing is common and sensitivity to sunlight is particularly high. Accordingly, rosacea may represent a type of hypersensitivity reaction disease in which vascular sensitivity is a central mechanism in its etiology. The correlation between sensitive blood vessels and sensitive skin has, however, not yet been determined. Epidemiological studies suggest that the regulatory mechanism of blood vessels may be of importance in the onset and development of rosacea. Studies show that 27% of rosacea patients were found to suffer from migraine and 42% from a tendency to flush, both of which represent about twice the level that would typically be found in a control group.

Psoriasis is a chronic, widespread skin disorder afflicting millions of humans and even domesticated animals. The disorder is characterized by recurrent, elevated red lesions, plaques or rarely pustules on the skin. These plaques are the results of an excessively rapid growth and shedding of epidermal (skin) cells. The cause of psoriasis is unknown.

Eczema (including but not limited to atopic, nummular and hand types) often has similar overlapping features with psoriasis. See, e.g., H. Roenigk, Jr. et al., "Psoriasis", ©1991, Marcel Dekker, Inc., Chapter 2. For instance, it is often difficult to distinguish based on clinical appearance. They can coexist, or the. disease can begin as eczema and over time turn to psoriasis. Again, treatments are similar with corticosteroids and preparations commonly employed for both of these conditions.

Another skin condition commonly confined to the scalp is seborrheic dermatitis (seborrhea). The least severe form, but most common, is simple dandruff. It can become more severe and form scaly, red patches on the face, ears, chest, and other widespread areas. It often coexists with psoriasis, and many subjects have overlapping features termed "seborrhiasis." Therefore, a continuum may exist whereby these are on the same disease spectrum. Treatments are similar to those currently used for psoriasis, although lower dosages are usually sufficient to control seborrheic dermatitis.

Inflammatory skin diseases refer to diseases that are accompanied by a series of clinical signs and symptoms, such as itch, edema, erythema and abrasion are induced by various stimulative factors that cause a series of inflammatory reactions in the skin epithelium.

Seborrheic dermatitis is a dermatitis that frequently occurs on areas with a high sebum secretion, such as the scalp, the forehead and the armpit, and is also called seborrheic eczema. It causes much erythema and fine scale (dandruff) and often appears in persons in the 20-40 age group. Unlike common eczema, it is a disease resulting from abnormal constitution or sebum secretion, and is characterized in that it causes the skin to be sensitive to sunlight or heat, grows worse mainly in spring and autumn and tends to recur.

In addition to the above-described conditions, aging of the skin is a complex phenomenon that can result in skin changes that may take a variety of different forms. Skin aging can result from cigarette smoke, chemicals, ultraviolet radiation. In particular, UV radiation has led to increased prevalence of photoaging. Photoaging can be prevented by sun avoidance and proper skin protection. Skin conditions that may occur as skin ages include dry skin, zerosis, ichthyosis, brownish spots, keratoses, melasma, lentigines, age spots, liver spots, pigmented spots, wrinkles, skin lines, fine lines, oily akin, warts, eczema, pruritic skin, psoriasis, inflammatory dermatoses, or disturbed keratinization.

While there are many compounds described in the art useful for improving the appearance and condition of skin, there is still a need for compositions with improved efficacy without undesirable side effects. The present invention relates to cosmetic methods of improving skin appearance and/or condition by topical application of the subject compositions.

[D1] JP 2000/063894 discloses a detergent composition for automatic dishwashers which is based on a high-alkaline sodium hydroxide solution, the pH of which is 9-11 in a 1 wt.% aqueous solution. An example of a composition is given which includes the ingredients sodium metasilicate, sodium carbonate, sodium gluconate, and sodium sulfate as the balance.

[D2] EP 0 441 057 A discloses detergent compositions which may contain metasilicates, carbonates, sulfates and gluconates as additional builders and sequestering agents.

[D3] US 2005/0097683 A1 discloses a water-free composition for bleaching and brightening human hair at the same time with a simultaneous conditioning effect. In Example 5 a dust-free bleaching powder is described having no less than 13 ingredients next to Coenzyme Q 10 and cetyl PG hydroxyethyl palmitamide which includes sodium carbonate, sodium metasilicate and sodium lauryl sulfate.

[D4] GB 1 367 359 A discloses a mineral food supplement in tablet form for the treatment of lack of minerals comprising substantially neutral physiologically acceptable readily dissociable mineral salts and a scandium salt, and preferably also an antimony salt. The composition may further include a) sodium metasilicate, b) lithium carbonate, c) calcium gluconate and ferrous gluconate, and d) aluminium potassium sulfate, zinc sulfate and cupric sulfate.

[D5] EP 0 217 975 A1 and its U.S. counterpart US 4,943,432, disclose a salt composition for use in the treatment of psoriasis. The salt mixture contains many of the salts which are naturally present as components of Dead Sea water, but it is free from organic impurities which are found in said water, like bitumens and oil tars. The mixture is mixture is primarily based on magnesium chloride, sodium, calcium and potassium chloride and comprise at least nine different cations and nine different anions including sulfate, silicate and carbonate.

**[D6]** JP 3 227378 B2 describes the use of silicic acid derivatives, e.g. metasilicic acid as an epidermal cornification accelerator for the treatment of atopic dermatitis, psoriasis, and the like.

[D7] WO 00/13649, which corresponds with EP 1 107 766 B1, discloses the use of lithium gluconate for making a medicine for treating of herpes and sebhorreic dermatitis, preferably in a composition designed for topical administration.

[D8] DE 37 05 894 A1 discloses tamoxifen gluconate for the treatment of psoriasis.

### BRIEF SUMMARY OF THE INVENTION

The present invention relates to compositions for the treatment of human skin, particularly facial skin and the scalp. Where the invention is used for the treatment of skin including the scalp, the compositions are useful for imparting a visual improvement in skin appearance, for alleviating the symptoms of cosmetic or dermatologic skin conditions, or for alleviating conditions of the scalp such as dandruff.

The present invention is directed to a composition for use in treating skin damage as defined in claim 1. Specific and preferred embodiments are defined in dependent claims 2-13. The invention is also directed to the use of such a composition for the preparation of a medicament for the treatment of skin damage as defined in claim 14.

Topical carriers include powders, lotions, gels, sprays, sticks, creams, ointments, liquids, emulsions, foams and aerosols. The compositions may also be used in combination with a fragrance, an anti-microbial such as a bactericide or fungicide, acne medications, wart remover such as salicylic acid with or without other hydroxyl acids, a reductant to bleach skin spots such as hydroxyquinone, a nutrient such as vitamin A or other vitamins and sunscreens. The composition may also be incorporated into a cosmetic, night cream, or skin ointment. Further, the composition may be used with lip balm, hand creams or lotions, bath salts or other bath additives, and treatments for hands, feet, nails, lips or the skin surrounding the eye area.

In one embodiment, the composition further comprises another cosmetically active agent. What is meant by a "cosmetically active agent" is a compound that has a cosmetic or therapeutic effect on the skin, e.g., agents to treat wrinkles, acne, or to lighten the skin. In one embodiment, the agent is selected, but not limited to, from the group consisting of hydroxy acids, benzoyl peroxide, sulfur resorcinol, ascorbic acid, D-panthenol, hydroquinone, sunscreen agents, anti-inflammatory agents, skin lightening agents, antimicrobial and antifungal agents, estrogens, 2-dimethylaminoethanol, lipoic acid, amino acids such a proline and tyrosine, lactobionic acid, acetyl-coenzyme A, niacin, riboflavin, thiamin, ribose, electron transporters such as NADH and FADH2, botanical extracts such as aloe vera and soy, and derivatives and mixtures thereof. The cosmetically active agent will typically be present in the composition of the invention in an amount of from about 0.001% to about 20% by weight of the composition, e.g., about 0.01% to about 10%, e.g., about 0.1% to about 5%.

Examples of hydroxy acids include, but are not limited, to (i) alpha-hydroxy acids such as glycolic acid, lactic acid, malic acid, citric acid, and tartaric acid; (ii) beta-hydroxy acids such as salicylic acid; and/or (iii) polyhydroxy acids. See, e.g., European Patent Application No. 273,202.

Examples of derivatives of ascorbic acid include, but are not limited to, ascorbyl palmitate, magnesium ascorbyl phosphate, sodium ascorbyl phosphate, zinc ascorbyl phosphate, ascorbyl glucoside, sodium ascorbate, and ascorbyl polypeptide. An example of a derivative of hydroquinone includes, but is not limited to, arbutin.

### DETAILED DESCRIPTION OF THE INVENTION

All percentages and ratios used herein are by weight of the total composition and all measurements made are at 25°C unless otherwise noted. All percentages, parts and ratios are based upon the total weight of the compositions of the present invention, unless otherwise specified. All such weights as they pertain to listed ingredients are based on the active level and, therefore, do not include solvents or by-products that may be included in commercially available materials, unless otherwise specified.

One skilled in the art will readily appreciate that the present invention is well adapted to carry out the objects and obtain the ends and advantages mentioned as well as those inherent therein. It should be understood, however, that the materials, compounds, coatings, methods, procedures, and techniques described herein are presently representative of embodiments. These techniques are intended to be exemplary, are given by way of illustration only, and are not intended as limitations on the scope.

As would be known to one of ordinary skill in the art, many variations of nomenclature are commonly used to refer to a specific chemical composition. Accordingly, several common alternative names may be provided herein in quotations and parentheses/brackets, or other grammatical technique, adjacent to a chemical composition's preferred designation when referred to herein. Additionally, many chemical compositions referred to herein are further identified by a Chemical Abstracts Service registration number. As would be known to those of ordinary skill in the art, the Chemical Abstracts Service provides a unique numeric designation, denoted herein as "CAS No.," for specific chemicals and some chemical mixtures, which unambiguously identifies a chemical composition's molecular structure.

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, representative embodiments of methods, devices, and materials are now described. Nothing herein is to be construed as an admission that the invention is not entitled to antedate such disclosure by virtue of prior invention.

Active and other ingredients useful herein may be categorized or described herein by their cosmetic and/or therapeutic benefit or their postulated mode of action. However, it is to be understood that the active and other ingredients useful herein can in some instances provide more than one cosmetic and/or therapeutic benefit or operate via more than one mode of action. Therefore, classifications herein are made for the sake of convenience and are not intended to limit an ingredient to the particularly stated application or applications listed.

### Definitions

In order to provide a clear and consistent understanding of the specification and claims, including the scope to be given such terms, the following definitions are provided:

As used herein other than the claims, the terms "a," "an," "the," and "the" means one or more. As used herein in the claim(s), when used in conjunction with the words "comprises" or "comprising," the words "a," "an," "the," or "the" may mean one or more than one. As used herein "another" may mean at least a second or more.

As used herein, the term "about" modifying the quantity of an ingredient in the compositions of the invention or employed in the methods of the invention refers to variation in the numerical quantity that can occur, for example, through typical measuring and liquid handling procedures used for making concentrates or use compositions in the real world; through inadvertent error in these procedures; through differences in the manufacture, source, or purity of the ingredients employed to make the compositions or carry out the methods; and the like. Whether or not modified by the term "about", it is intended that the claims include equivalents to the quantities.

As used herein, the term "benefit agent" includes any active ingredient that is to be delivered into and/or onto the skin, hair or nail at a desired location, such as a cosmetic agent or a pharmaceutical agent. By "cosmetic agent," it is meant any ingredient that is appropriate for cosmetically treating, providing nutrients to, and/or conditioning the hair, nail, and/or skin via topical application. By "pharmaceutical agent," it is mean any drug that is either hydrophobic or hydrophilic in nature and appropriate for topical use. As used herein "medicament agents" include those agents capable of promoting recovery from injury and illness.

As used herein, the term "cosmetics" includes make-up, foundation, and skin care products. The term "make-up" refers to products that leave color on the face, including foundation, blacks and browns, i.e., mascara, concealers, eye liners, brow colors, eye shadows, blushers, lip colors, powders, solid emulsion compact, and so forth. "Skin care products" are those used to treat or care for, or somehow moisturize, improve, or clean the skin. Products contemplated by the phrase "skin care products" include, but are not limited to, adhesives, bandages, toothpaste, anhydrous occlusive moisturizers, antiperspirants, deodorants, personal cleansing products, powder laundry detergent, fabric softener towels, occlusive drug delivery patches, nail polish, powders, tissues, wipes, hair conditioners-anhydrous, shaving creams and the like. The term "foundation" refers to liquid, cream, mousse, pancake, compact, concealer or like product created or reintroduced by cosmetic companies to even out the overall coloring of the skin.

The term "dermatologically-acceptable" or "cosmetically-acceptable" as used herein, means that the compounds or composition(s) which the term describes are suitable for use in contact with tissues (e.g., the skin) without undue toxicity, incompatibility, instability, irritation, allergic response and the like. This term is not intended to limit the compound/composition to which it describes for use solely as a cosmetic (i.e., the ingredient/product may be used as a pharmaceutical).

As used herein, "relief of symptoms" refers to decrease in severity over that expected in the absence of treatment and does not necessarily indicate a total elimination or cure of the disease. Relief of symptoms is also intended to include prophylaxis.

As used herein, "topically applying" means directly laying on or spreading on outer skin, e.g., by use of hands or an applicator such as a wipe, puff, roller or spray.

As used herein, the term "safe and effective amount" means an amount of compound(s) or composition(s) sufficient to treat acne or other skin conditions described herein, but low enough to avoid serious side effects, i.e., to provide a reasonable benefit-to-risk ratio, within the scope of sound medical judgment.

As used herein, the term "treating" or "treatment" means the treatment (e.g., alleviation or elimination of symptoms and/or cure) and/or prevention or inhibition of the condition (e.g. a skin condition) or relief of symptoms.

### Compositions

The present invention relates to compositions and their use for the treatment of skin damage, particularly facial skin, or the scalp. The present invention is directed to a conditioning composition comprising (a) one or more metasilicate; (b) one or more carbonate; (c) one or more gluconate; and (d) one or more sulfate which includes potassium aluminium sulfate. The composition may also contain (e) salts, e.g., sea salts, and other additives. The composition may also include other active ingredients.

### Metasilicate

The present invention may include one or more metasilicate which may be an alkali metal silicate selected from the group consisting of sodium or potassium metasilicate, sodium or potassium orthosilicate and mixtures thereof. In one embodiment, the metasilicate is incorporated in the final composition in an amount of 0.01%-5.0%, or 0.1-4.0%, based on the total amount of the composition.

### Carbonate

In another embodiment, the carbonate used in the present invention is one or more carbonates selected from the group consisting of sodium carbonate, potassium carbonate, sodium bicarbonate, potassium bicarbonate, and sodium sesquicarbonate. In another embodiment, the carbonate used in the present invention is sodium carbonate and sodium bicarbonate. In another embodiment, the carbonates is incorporated in the final composition in an amount of 0.01%-4.0%, or 0.1-3.0%, based on the total amount of the composition.

Typical carbonates include sodium carbonate (Na₂CO₃), potassium carbonate (K₂CO₃) or other typical carbonate sources. Such carbonates can contain as an impurity some proportion of bicarbonate (HCO₃).

In one embodiment, the composition further comprises peroxygen compound. The peroxygen compound is preferably a perborate or a percarbonate and more preferably a percarbonate. The perborate or percarbonate preferably is complexed with a metal such as sodium, lithium, calcium, potassium or boron. The percent by weight of the peroxygen compound in the final composition ranges from about 0.01%-4.0%, or 0.1-3.0%, based on the total amount of the composition.

In another embodiment, the carbonate is a builder wherein the builder is at least one of the following compoundsi a sodium carbonate (e.g., soda ash), sodium sesquicarbonate, sodium sulfate or sodium bicarbonate. A builder is also known as a sequestrant. A "sequestrant" is a molecule capable of coordinating (i.e., binding) metal ions to prevent the metal ions from interfering with the action of the other ingredients of the composition. Some chelating/sequestering agents can also function as a threshold agent when included in an effective amount. Optionally, the builders can be added, e.g., water soluble inorganic salt builders, preferably sodium salts, such as sodium polyphosphates, e.g. sodium tripolyphosphate and sodium pyrophosphate, sodium carbonate, sodium bicarbonate, sodium sesquicarbonate, sodium silicate, sodium disilicate, sodium metasilicate and sodium borate. In addition to the water soluble inorganic salts, water insoluble builders may also be useful, including the ion exchanging zeolites, such as Zeolite 4A. Organic builders may also be employed. Among suitable organic builders are polyacetal carboxylates, as described in U.S. Pat. No. 4,725,455, and water-soluble salts of lower hydroxycarboxylic acids, such as an alkali metal gluconate.

In one embodiment, the carbonate is a hydrated carbonate such as trona. In one embodiment, the percent by weight of the builder is from about 0.01%-4.0%, or 0.1-3.0%, based on the total amount of the composition. In another embodiment, the peroxygen compound, metasilicate and chelate are all salts having the same cation. In one embodiment, the cation is sodium or potassium.

### Gluconate

In one embodiment, the gluconate is selected from the group consisting of ammonium gluconate, lithium gluconate, sodium gluconate, sodium starch gluconate, potassium gluconate, ammonium acid gluconate, sodium acid gluconate, lithium acid gluconate, potassium acid gluconate, ammonium D-gluconate, lithium D-gluconate, sodium D-gluconate, potassium D-gluconate, glyconic acid, glyconic D acid, glyconic L acid, ammonium L-gluconate, lithium L-gluconate, sodium L-gluconate, potassium L-gluconate, magnesium gluconate, magnesium acid gluconate, magnesium D-gluconate, magnesium L-gluconate, calcium gluconate, calcium acid gluconate, calcium D-gluconate, calcium L-gluconate and mixtures thereof.

In one embodiment, the percent by weight of the builder is from about 0.001%-0.4%, or 0.01-0.3%, based on the total amount of the composition.

### Sulfate or Aluminium Salts

Examples of aluminium salts suitable for use in the present invention include inorganic aluminium salts such as potassium aluminium sulfate, ammonium aluminium sulfate and aluminium chloride; and soluble aluminium carboxylates such as aluminium lactate, aluminium citrate and aluminium maleate.

Regarding these aluminium salts, in one embodiment, at least 90% by weight (hereinafter referred to as "%") or more of their particles have diameters of 200 micrometers or less. In one embodiment, at least 90% or more of the particles which make up the composition have diameters of 200 micrometers or less, and, in one embodiment, the average particle diameter falls within a range of 20-150. Ideally, the average particle diameter should be between 20 and 100 micrometers. These aluminium salts can be used singly or in combination.

### Salts

In one embodiment, the composition contains a salt. The salt should not interfere with the biological activity of the composition. When other materials are present, the salt should not degrade those materials or interfere with their properties or biological activity. In other words, the salt should be inert with respect to the other components.

The salt may be a single salt material or a mixture of two or more salts alone. When the carrier matrix contains a mixture of salts, those salts are preferably present in equal amounts, e.g., a mixture of two salts in a 1:1 ratio.

Examples of suitable salts include various alkali metal and/or alkaline earth metal sulfates, chlorides, borates, bromides, citrates, acetates, lactates, etc. Specific examples of suitable salts include, but are not limited to, sodium acetate, sodium bicarbonate, sodium borate, sodium bromide, sodium carbonate, sodium chloride, sodium citrate, sodium fluoride, sodium gluconate, sodium sulfate, calcium chloride, calcium lactate, calcium sulfate, potassium sulfate, tripotassium phosphate, potassium chloride, potassium bromide, potassium fluoride, magnesium chloride, magnesium sulfate and lithium chloride. The preferred salts are the inorganic salts, such as the Group 1 or 2 metal sulfates and chlorides. Particularly preferred salts, because of their low cost, are sodium sulfate, and sodium chloride. Sodium chloride may be substantially pure or in the form of rock salt, sea salt, or dendrite salt.

In another embodiment, the composition, prior to final use, is prepared as a composition comprising:

| | |
|---|---|
| Meta Silicate | about 0.7 - 3.3 %wt |
| Carbonate | about 0.7 - 1.7 % wt |
| Gluconate | about 0.33- 1.7 % wt |
| Aluminium Sulfate | about 0.33 - 1.7 % wt |

In another embodiment, the composition, prior to final use, is prepared as a composition comprising:

| | |
|---|---|
| Meta Silicate | about 1.33- 2.0% wt |
| Carbonate | about 1.33 - 1.7% wt |
| Gluconate | about 0.33 - 0.7 % wt |
| Potassium Al Sulfate | about 0.33 - 0.7 % wt |

In another embodiment, the composition, prior to final use, is prepared as a composition comprising:

| | |
|---|---|
| MetaSilicate | about 0.7-3.3 % wt |
| Carbonate | about 0.7-3.3 % wt |
| Gluconate | about 0.33-1.7 % wt |
| Potassium Al Sulfate | about 0.33-1.7 % wt |
| Inorganic Salt | about 0.33-1.7% wt |

In another embodiment, the composition, prior to final use, is prepared as a composition comprising:

| | |
|---|---|
| Meta Silicate | about 1.3 - 2.0% wt |
| Carbonate | about 1.3- 1.7% wt |
| Gluconate | about 0.3-0.7% wt |
| Potassium Al Sulfate | about 0.3-0.7 % wt |
| Inorganic Salt | about 0.3-0.7 % wt |

In another embodiment, the composition, prior to final use, is prepared as a composition comprising:

| | |
|---|---|
| Meta Silicate | about 1.63 % wt |
| Sodium Carbonate | about 1.5 % wt |
| Sodium Gluconate | about 0.5 % wt |
| Potassium Al Sulfate | about 0.5 % wt |

In another embodiment, the composition, prior to final use, is prepared as a composition comprising:

| | |
|---|---|
| Meta Silicate | about 1.63 % wt |
| Sodium Carbonate | about 1.5 % wt |
| Sodium Gluconate | about 0.5 % wt |
| Inorganic salt | about 0.5 % wt |
| Potassium Al Sulfate | about 0.5 % wt |

In one specific embodiment, the invention provides for a composition comprising:
(a) at least 0.0001 % wt of one or more metasilicates;
(b) at least 0.0001 % wt of one or more carbonate
(c) at least 0.00005 % wt of one or more gluconate; and
(d) at least 0.00002 % wt of one or more sulfate selected from the group consisting of potassium aluminium sulfate, sulfuric acid, sodium sulfate, potassium sulfate, lithium sulfate, ammonium sulfate, magnesium sulfate, strontium sulfate, and aluminium sulfate;
   wherein the concentrations are the concentration in final composition.

In another specific embodiment, the present invention provides for a composition additionally comprising:
(e) at least 0.00006 % wt of one or more salts.

In one specific embodiment, the present invention provides for a composition comprising:
(a) at least 0.0001 % wt of one or more metasilicates;
(b) at least 0.0002 % wt of one or more carbonate
(c) at least 0.00008 % wt of one or more gluconate; and
(d) at least 0.00008 % wt of one or more sulfate selected from the group consisting of potassium aluminium sulfate, sulfuric acid, sodium sulfate, potassium sulfate, lithium sulfate, ammonium sulfate, magnesium sulfate, strontium sulfate, and aluminium sulfate;
wherein the concentrations are the concentration in the final composition.

In another specific embodiment, the present invention provides for a composition additionally comprising:
(e) at least 0.0001 % wt of one or more salts.

In one specific embodiment, the present invention provides for a composition comprising:
(a) at least 0.0003 % wt of one or more alkali metal silicate selected from the group consisting of sodium or potassium metasilicate, orthosilicate or other water-soluble silicate;
(b) at least 0.0003 % wt of one or more carbonate selected from the group consisting of sodium carbonate, sodium sesquicarbonate, sodium sulfate and sodium bicarbonate;
(c) at least 0.00009 % wt of one or more gluconate; and
(d) at least 0.00008 % wt of one or more sulfate selected from the group consisting of potassium aluminium sulfate, sulfuric acid, sodium sulfate, potassium sulfate, lithium sulfate, ammonium sulfate, magnesium sulfate, strontium sulfate, and aluminium sulfate;
wherein the concentrations are the concentration in final composition.

In another specific embodiment, the present invention provides for a composition additionally comprising:
(e) at least 0.00006 % wt of one or more salts.

In one specific embodiment, the present invention provides for a composition comprising:
(a) at least 0.0001 % wt of metasilicates;
(b) at least 0.0002 % wt of sodium carbonate
(c) at least 0.00008 % wt of sodium gluconate; and
(d) at least 0.00008 % wt of potassium aluminium sulfate;
wherein the concentrations are the concentration in the final composition.

In another specific embodiment, the present invention provides for a composition additionally comprising:
(e) at least 0.0001 % wt of one or more salts.

In one specific embodiment, the present invention provides for a composition comprising:
(a) from about 0.0001 to about 0.0100 % wt of one or more metasilicates;
(b) from about 0.0001 to about 0.0100 % wt of one or more
(c) from about 0.00001 to about 0.0060 % wt of one or more gluconate; and
(d) from about 0.00001 to about 0.0100 % wt one or more sulfate,
wherein the concentrations are the concentration in final composition.

In another specific embodiment, the present invention provides for a composition additionally comprising:
(e) from about 0. 0001 to about 0.0100 % wt of one or more salts.

In one specific embodiment, the present invention provides for a composition comprising:
(a) from about 0.0001 to about 0.0010 % wt of one or more metasilicates;
(b) from about 0.0001 to about 0.0010 % wt of one or more carbonate
(c) from about 0.000001 to about 0.0006 % wt of one or more gluconate; and
(d) from about 0.0001 to about 0.0010 % wt of potassium aluminium sulfate,
wherein the concentrations are the concentration in final composition.

In another specific embodiment, the present invention provides for a composition additionally comprising:
(e) from about 0.0001 to about 0.0010 % wt of one or more salts.

The substances to be used in the compositions of the present invention are generally utilized within the following ranges:

**Table 1**

| Substance | % Weight of Total Composition | | |
|---|---|---|---|
| | | | |
| Meta Silicate | 0.01 | - | 10.0 |
| Sodium Carbonate | 0.01 | - | 8.50 |
| Sodium Gluconate | 0.00001 | - | 5.40 |
| Sea salt | 0.01 | - | 9.20 |
| Potassium Al Sulfate | 0.00015 | - | 0.0830 |
| Meta Silicate | 0.011 | - | 5.0 |
| Sodium Carbonate | 0.017 | - | 7.20 |
| Sodium Gluconate | 0.005 | - | 4.20 |
| Sea salt | 0.006 | - | 3.00 |
| Potassium Al Sulfate | 0.009 | - | 2.75 |
| Meta Silicate | 0.012 | - | 0.07 |
| Sodium Carbonate | 0.029 | - | 0.048 |
| Sodium Gluconate | 0.008 | - | 0.035 |
| Sea salt | 0.006 | - | 0.028 |
| Potassium Al Sulfate | 0.002 | - | 0.0190 |
| Meta Silicate | 0.035 | - | 0.065 |
| Sodium Carbonate | 0.032 | - | 0.04 |
| Sodium Gluconate | 0.009 | - | 0.014 |
| Sea salt | 0.01 | - | 0.0135 |
| Potassium Al Sulfate | 0.008 | - | 0.0135 |
| Fragrances | 0.01 | - | 0.011 |

**Table 2. Ranges Specific to Particular Embodiments**

| Composition | % Weight of Total Composition | | |
|---|---|---|---|
| Cosmetics/Skin Care Products | | | |
| Meta Silicate | 0.21 | - | 0.42 |
| Sodium Carbonate | 0.16 | - | 0.32 |
| Sodium Gluconate | 0.06 | - | 0.13 |
| Potassium Al Sulfate | 0.06 | - | 0.13 |
| Meta Silicate | 1 | - | 5 |
| Sodium Carbonate | 0.5 | - | 5 |
| Sodium Gluconate | 0.1 | - | 2 |
| Potassium Al Sulfate | 0.1 | - | 2 |
| Meta Silicate | 2 | - | 10 |
| Sodium Carbonate | 1 | - | 10 |
| Sodium Gluconate | 0.5 | - | 5 |
| Potassium Al Sulfate | 0.5 | - | 5 |

### Uses of the Present Invention related to Skincare

The topical formulations of the invention contain the metasilicate, carbonate, gluconate, and sulfate ingredients in a concentration effective to prevent or reduce (hereafter, "inhibit") the skin irritation (such as itching, dry skin, inflammation) symptoms that are sought to be eliminated. In one embodiment, the formulation contains the ingredients in a suitable topical vehicle at a total concentration of about 10 to about 5000 mM. In another embodiment, the formulation contains the ingredients in a suitable topical vehicle at a total concentration of about 25 to about 3000 mM. In another embodiment, the formulation contains the ingredients in a suitable topical vehicle at a total concentration of about 50 to about 2000 mM. In another embodiment, the formulation contains the ingredients in a suitable topical vehicle at a total concentration of about 100 to about 1000 mM. In another embodiment, the formulation contains the ingredients in a suitable topical vehicle at a total concentration of about 200 to about 500 mM. In another embodiment, the formulation of the invention includes additional ingredients such as an exfoliant ingredient.

The compositions and methods of the present invention are useful for treating follicular diseases such as acne, rosacea, hyperlipidemia, seborrhea, sebaceous hyperplasia, follicular rash, demodex folliculorum follicular infections such as folliculitis, staphylococcoal impetigoacne necrotica, and pseudofollicuitis barbe, follicular ketarosis, keratosis pilaris, phrynoderma, ichthyosis follicularis, alopecia, follicular dysplasia, hirsutism, oily skin and hypertrichosis. In one embodiment, the present invention relates to topical compositions that may also include an anti-acne agent. Examples of anti-acne agents include, but are not limited to, salicylic acid, benzoyl peroxide, sulphur, retinoic acid, candida bombicola/glucose/methyl rapeseedate ferment, peat water, resorcinol, silt, peat, permethin, azelaic acid, clindamycin, adapalene, erythromycin, sodium sulfacetamide, and combinations thereof. In one embodiment, the amount of anti-acne agent in the composition is from about 0.01% to about 10%, for example from about 0.1% to about 5% or from about 0.5% to about 2% by weight, based on the total weight of the composition.

The compositions and methods of the present invention are also useful for evening skin tone (such as lightening dark areas of skin) in need of such treatment, smoothing the skin (such as reducing texture on the skin), reducing the production of sebum, and reducing the appearance of oil, shine, and/or pores on skin in need of such treatment. Examples of skin in need of such treatment include, but are not limited to, skin having excessive pigmentation (such as freckles, post-inflammatory hyperpigmentation (PIET), or pigmented scars), rough skin, oily skin or skin having large, visible pores. In another embodiment, the present invention relates to topical compositions that may also include other active ingredients known to improve skin condition or appearance.

The compositions and methods of the present invention are also useful for the treatment of skin conditions such as eczema. In one embodiment, the present invention relates to topical compositions that may also include other active ingredients known to improve or treat eczema. In other embodiments, the present invention may be used for th treatment of rosacea. In yet another embodiment, the present invention may also include one or more anti-rosacea agents.

In one embodiment, the composition is heated prior to application. In one embodiment, the temperature of the composition should not exceed a temperature of 100° F.

In one embodiment, the present invention is directed to a cosmetic composition and corresponding methods of applying the composition directly to the skin or scalp, wherein the composition comprises from about 0.1% to about 50% by weight of the conditioning composition and from about 50% to about 99.9% by weight of a suitable carrier.

In one embodiment, the present invention is directed to a cosmetic composition and corresponding methods of applying the composition directly to the skin or scalp, wherein the composition comprises from about 0.1% to about 40% by weight of the conditioning composition, from about 0.005% to about 20% by weight of a salt, and from about 40% to about 99% by weight of a suitable carrier.

In one embodiment, the present invention is directed to a cosmetic composition and corresponding methods of applying the composition directly to the skin or scalp, wherein the composition comprises from about 0.1% to about 20% by weight of the conditioning composition, from about 0.005% to about 20% by weight of a benefit agent, and from about 60% to about 99% by weight of a suitable carrier.

In another embodiment, the composition is a leave-on cosmetic composition and corresponding methods of applying the composition directly to the scalp, wherein the composition comprises from about 0.1% to about 20% by weight of the conditioning composition, from about 0.005% to about 20% by weight of a moisterizing agent or carrier, and from about 40% to about 99% by weight of a volatile liquid.

In another embodiment, the composition further comprises a thickening polymer in an amount of 0.01 to 20 wt % based on the weight of the conditioning composition.

In another embodiment, the composition further comprises one or more of at least one amphoteric surfactant; at least one nonionic surfactant or at least one phospholipid; and at least one wax; wherein the ingredients are present in a combined amount sufficient to allow the conditioning composition to be incorporated into an aqueous solution

In another embodiment, the composition further comprises a fatty alcohol present in an amount of from about 2.5% to about 4% by weight of the composition.

In another embodiment, the composition further comprises water present in an amount of from about 60% to about 96% by weight of the composition.

In another embodiment, the composition further comprises at least one of the following additives: a thickener, a dye, a fragrance, and/or a preservative.

### Benefit Agents

The compositions of the present invention may be used as a skin conditioning system. The skin conditioning system may further contain one or more benefit agents or pharmaceutically-acceptable salts thereof. The benefit agents useful herein may be categorized by their therapeutic benefit or their postulated mode of action. However, it is to be understood that the benefit agents useful herein may, in some circumstances, provide more than one therapeutic benefit or operate via greater than one mode of action. Therefore, the particular classifications provided herein are made for the sake of convenience and are not intended to limit the benefit agents to the particular application(s) listed. In addition, the compounds, which are identified below as being suitable for use as benefit agents, may be used in an amount over and above the amount that they may be used for other purposes in the conditioning system.

Examples of suitable benefit agents include, but are not limited to, depigmentation agents; reflectants; detangling/wet combing agents; film forming polymers; humectants; amino acids and their derivatives; antimicrobial agents; allergy inhibitors; anti-acne agents; anti-aging agents; anti-wrinkling agents antiseptics; analgesics; antifussives; antipruritics; local anesthetics; anti-hair loss agents; hair growth promoting agents; hair growth inhibitor agents, antihistamines such as Mandragora Vernalis, Tanacetum Parthenium and the like; antiinfectives such as Acacia Catechu, Aloe Barbadensis, Convallaria Majalis, Echinacea, Eucalyptus, Mentha Piperita, Rosa Canina, Sassafras Albidum, and the like; inflammation inhibitors; anti-emetics; anticholinergics; vasoconstrictors; vasodilators; wound healing promoters; peptides, polypeptides and proteins; deodorants and antiperspirants; medicament agents; skin emollients and skin moisturizers; skin firming agents, hair conditioners; hair softeners; hair moisturizers; vitamins; tanning agents; skin lightening agents; antifungals such as Centaurea Cyanus, Kalmia Latifolia and antifungals for foot preparations; depilating agents; shaving preparations, external analgesics; perfumes; counterirritants; hemorrhoidals; insecticides, poison ivy products; poison oak products; burn products; anti-diaper rash agents; prickly heat agents; make-up preparations; vitamins; amino acids and their derivatives; herbal extracts; retinoids; flavenoids; sensates; anti-oxidants; skin conditioners; hair lighteners; chelating agents; cell turnover enhancers; coloring agents; pigments; sunscreens, those active ingredients disclosed in U.S. Pat. No. 6,063,397, anti-edema agents, collagen enhancers, and mixtures thereof.

Examples of suitable anti-edema agents nonexclusively include bisabolol natural, synthetic bisabolol, and mixtures thereof.

Examples of suitable vasoconstrictors nonexclusively include horse chestnut extract, prickly ash, and mixtures thereof.

Examples of suitable anti-inflammatory agents nonexclusively include benoxaprofen, centella asiatica, bisabolol, feverfew (whole), feverfew (parthenolide free), green tea extract, green tea concentrate, hydrogen peroxide, lycopene, oat oil, chamomile, and mixtures thereof.

Examples of collagen enhancers nonexclusively include vitamin A, vitamin C, and mixtures thereof.

Examples of suitable skin firming agent nonexclusively include dimethylaminoethanol ("DMAE").

Examples of suitable antipruritics and skin protectants nonexclusively include oatmeal, betaglucan, feverfew, soy and derivatives thereof, bicarbonate of soda, colloidal oatmeal, surfactant based colloidal oatmeal cleanser, Anagallis Arvensis, Oenothera Biennis, Verbena Officinalis, and the like. These antipruritics may be used in an amount, based upon the total weight of the skin conditioning composition, from about 0.01 percent to about 40 percent, and preferably from about 1 percent to about 5 percent.

As used herein, colloidal oatmeal means the powder resulting from the grinding and further processing of whole oat grain meeting United States Standards for Number 1 or Number 2 oats. The colloidal oatmeal has a particle size distribution as follows: not more than 3 percent of the total particles exceed 150 micrometers in size and not more than 20 percent of the total particles exceed 75 micrometers in size.

Examples of suitable reflectants nonexclusively include mica, alumina, calcium silicate, glycol dioleate, glycol distearate, silica, sodium magnesium fluorosilicate, and mixtures thereof.

Suitable detangling/wet combing agents nonexclusively include polyquaternium-10, hydroxypropyltrimonium guar, dioleoylamidoethyl hydroxyethylmonium methosulfate, di-(soyoylethyl) hydroxyethylmonium methosulfate, hydroxyethyl behenamidopropyl dimonium chloride, olealkonium chloride, polyquaternium-47, stearalkonium chloride, tricetylmonium chloride, and mixtures thereof.

Suitable film forming polymers include those that, upon drying, produce a substantially continuous coating or film on the heir, skin, or nails. Nonexclusive examples of suitable film forming polymers include acrylamidopropyl trimonium chloride/acrylamide copolymer; corn starch/acrylamide/sodium acrylate copolymer, polyquaternium-10; polyquaternium-47; polyvinylmethylether/maleic anhydride copolymer; styrene/acrylates copolymers; and mixtures thereof.

Commercially available humectants which are capable of providing moisturization and conditioning properties to the skin conditioning composition are suitable for use in the present invention. The humectant is preferably present in an amount of from about 0 percent to about 10 percent, more preferably from about 0.5 percent to about 5 percent, and most preferably from about 0.5 percent to about 3 percent, based on the overall weight of the composition. Examples of suitable humectants nonexclusively include: 1) water soluble liquid polyols selected from the group comprising glycerine, propylene glycol, hexylene glycol, butylene glycol, pentylene glycol, dipropylene glycol, and mixtures thereof; 2) polyalkylene glycol of the formula L: HO--(R"O)_{b}--H I. wherein R" is an alkylene group having from about 2 to about 4 carbon atoms and b is an integer of from about 1 to about 10, such as PEG 4; 3) polyethylene glycol ether of methyl glucose of formula II.: CH₃-C₆H₁₀O₅--(OCH₂CH₂)_{c}--OH II. wherein c is an integer from about 5 to about 25; 4) urea; 5) fructose; 6) glucose; 7) honey; 8) lactic acid; 9) maltose; 10) sodium glucuronate; and 11) mixtures thereof, with glycerine being the preferred humectant.

Suitable amino acid agents include amino acids derived from the hydrolysis of various proteins as well as the salts, esters, and acyl derivatives thereof. Examples of such amino acid agents nonexclusively include amphoteric amino acids such as alkylamido alkylamines, i.e. stearyl acetyl glutamate, capryloyl silk amino acid, capryloyl collagen amino acids; capryloyl keratin amino acids; capryloyl pea amino acids; cocodimonium hydroxypropyl silk amino acids; corn gluten amino acids; cysteine; glutamic acid; glycine; hair keratin amino acids; amino acids such as aspartic acid, threonine, serine, glutamic acid, proline, glycine, alanine, cystine, valine, methionine, isoleucine, leucine, tyrosine, phenylalanine, cysteic acid, lysine, histidine, arginine, cysteine, tryptophan, citrulline; lysine; silk amino acids, wheat amino acids; and mixtures thereof.

Suitable proteins include those polymers that have a long chain, i.e. at least about 10 carbon atoms, and a high molecular weight, i.e. at least about 1000, and are formed by self-condensation of amino acids. Nonexclusive examples of such proteins include collagen, deoxyribonuclease, iodized corn protein; milk protein; protease; serum protein; silk; sweet almond protein; wheat germ protein; wheat protein; alpha and beta helix of keratin proteins; hair proteins, such as intermediate filament proteins, high-sulfur proteins, ultrahigh-sulfur proteins, intermediate filament-associated proteins, high-tyrosine proteins, high-glycine tyrosine proteins, tricohyalin, and mixtures thereof.

In one embodiment, the composition further comprises a nutrient. What is meant by a nutrient is an organic substance occurring in foods that is not synthesized by the body and is necessary in trace amounts for the normal metabolic functioning of the body, such as vitamins, essential amino acids, and essential fatty acids.

Examples of suitable vitamins nonexclusively include vitamin B complex; including thiamine, nicotinic acid, biotin, pantothenic acid, choline, riboflavin, vitamin B6, vitamin B12, pyridoxine, inositol, camitine; vitamins A,C,D,E,K and their derivatives such as vitamin A palmitate and pro-vitamins, e.g. (i.e. panthenol (pro vitamin B5) and panthenol triacetate) and mixtures thereof.

Examples of such essential amino acids include, but are not limited to, arginine, histidine, isoleucine, leucine, lysine, methionine, phenylalanine, threonine, tryptophan, and valine. Examples of essential fatty acids include, but are not limited to, linoleate and linolenate.

Examples of suitable antibacterial agents nonexclusively include bacitracin, erythromycin, neomycin, tetracycline, chlortetracycline, benzethonium chloride, phenol, and mixtures thereof.

Examples of suitable external analgesics and local anesthetics nonexclusively include benzocaine, dibucaine, benzyl alcohol, camphor, capsaicin, capsicum, capsicum oleoresin, juniper tar, menthol, methyl nicotinate, methyl salicylate, phenol, resorcinol, turpentine oil, and mixtures thereof.

Examples of suitable antiperspirants and deodorants nonexclusively include aluminium chlorohydrates, aluminium zirconium chlorohydrates, and mixtures thereof.

Examples of suitable counterirritants nonexclusively include camphor, menthol, methyl salicylate, peppermint and clove oils, ichtammol, and mixtures thereof.

An example of a suitable inflammation inhibitor nonexclusively includes hydrocortisone, Fragaria Vesca, Matricaria Chamomilla, and Salvia Officinalis.

Examples of suitable hemorrhoidal products nonexclusively include the anesthetics such as benzocaine, pramoxine hydrochloride, and mixtures thereof; antseptics such as benzethonium chloride; astringents such as zinc oxide, bismuth subgallate, balsam Peru, and mixtures thereof; skin protectants such as cod liver oil, vegetable oil, and mixtures thereof.

Most preferred benefit agents nonexclusively include DMAE, soy and derivatives thereof, colloidal oatmeal, sulfonated shale oil, olive leaf, elubiol, 6-(1-piperidinyl)2,4-pyrimidinediamine-3-oxide, finasteride, ketoconazole, salicylic acid, zinc pyrithione, coal tar, benzoyl peroxide, selenium sulfide, hydrocortisone, sulfur, menthol, pramoxine hydrochloride, tricetylmonium chloride, polyquaternium 10, panthenol, panthenol triacetate, vitamin A and derivatives thereof, vitamin B and derivatives thereof, vitamin C and derivatives thereof, vitamin D and derivatives thereof, vitamin E and derivatives thereof, vitamin K and derivatives thereof, keratin, lysine, arginine, hydrolyzed wheat proteins, hydrolyzed silk proteins, octyl methoxycinnamate, oxybenzone, minoxidil, titanium dioxide, zinc dioxide, retinol, erthromycin, tretinoin, and mixtures thereof.

One preferred type of benefit agent includes those therapeutic components that are effective in the treatment of dandruff, seborrheic dermatitis, and psoriasis as well as the symptoms associated therewith. Examples of such suitable benefits agents nonexclusively include zinc pyrithione, anthralin, shale oil and derivatives thereof such as sulfonated shale oil, selenium sulfide, sulfur; salicylic acid; coal tar; povidone-iodine, imidazoles such as ketoconazole, dichlorophenyl imidazolodioxalan, which is commercially available from Janssen Pharmaceutica, N.V., under the tradename, "Elubiol", clotrimazole, itraconazole, miconazole, climbazole, tioconazole, sulconazole, butoconazole, fluconazole, miconazole nitrate and any possible stereo isomers and derivatives thereof; piroctone olamine (Octopirox); selenium sulfide; ciclopirox olamine; anti-psoriasis agents such as vitamin D analogs, e.g. calcipotriol, calcitriol, and tacaleitrol; vitamin A analogs such as esters of vitamin A, e.g. vitamin A palmitate, retinoids, retinols, and retinoic acid; corticosteroids such as hydrocortisone, clobetasone, butyrate, clobetasol propionate and mixtures thereof.

The amount of benefit agent to be combined with the skin conditioning composition or the emulsion may vary depending upon, for example, the ability of the benefit agent to penetrate through the skin, hair or nail, the specific benefit agent chosen, the particular benefit desired, the sensitivity of the user to the benefit agent, the health condition, age, and skin, hair, and/or nail condition of the user, and the like. In sum, the benefit agent is used in a "safe and effective amount," which is an amount that is high enough to deliver a desired skin, hair or nail benefit or to modify a certain condition to be treated, but is low enough to avoid serious side effects, at a reasonable risk to benefit ratio within the scope of sound medical judgment. Unless otherwise expressed herein, typically the benefit agent is present in the skin conditioning system in an amount, based upon the total weight of the system, from about 0.01 percent to about 20.0 percent, and preferably from about 0.01 percent to about 5.0 percent, and more preferably from about 0.01 percent to about 2.0 percent.

Optionally, commercially available detergent thickeners that are capable of imparting the appropriate viscosity to conditioning shampoo compositions are suitable for use in this invention. If used, the detergent thickeners should be present in the shampoo compositions in an amount sufficient to raise the Brookfield viscosity of the composition to a value of between about 500 to about 10,000 centipoise. Examples of suitable detergent thickeners nonexclusively include: mono or diesters of polyethylene glycol of formula IV. HO-(CH₂CH₂O)₂H IV. wherein z is an integer from about 3 to about 200; fatty acids containing from about 16 to about 22 carbon atoms; fatty acid esters of ethoxylated polyols; ethoxylated derivatives of mono and diesters of fatty acids and glycerine; hydroxyalkyl cellulose; alkyl cellulose; hydroxyalkyl alkyl cellulose; and mixtures thereof. More specifically, suitable detergent thickeners nonexclusively include behenalkonium chloride; cetyl alcohol, quaternium-46, hydroxyethyl cellulose, cocodimonium chloride, polyquaternium-6, polyquaternium-7, quaternium-18, PEG-18 glycerol oleate/cocoate, a mixture of acrylates/steareth-50 acrylate copolymer, laureth-3 and propylene glycol, which is commercially available from Goldschmidt under the tradename "Antil 208," a mixture of cocamidopropylbetaine and glyceryl laurate which is commercially available from Goldschmidt under the tradename, "Antil HS60," a mixture of propylene glycol, PEG 55, and propylene glycol oleate, which is commercially available from Goldschmidt under the tradename, "Antil 414 liquid," and mixtures thereof. Preferred detergent thickeners include polyethylene glycol ester, and more preferably PEG-150 distearate which is available from the Stepan Company of Northfield, Ill. or from Comiel, S.p.A. of Bologna, Italy under the tradename, "PEG 6000 DS".

The above described skin conditioning composition may be prepared by combining the desired components in a suitable container and mixing them under ambient conditions in any conventional mixing means well known in the art, such as a mechanically stirred propeller, paddle, and the like.

Another embodiment of the present invention is directed to a method for depositing a benefit agent onto the skin comprised of applying either the above-described skin conditioning system with an effective amount of a benefit agent to a desired location on a human or animal. While the frequency and amount of the benefit agent to be applied will depend upon, for example, the type and amount of benefit agent available, the intended usage of the final composition, i.e. therapeutic versus maintenance regimen, the amount and type of detergent present, and the sensitivity of the individual user to the composition/emulsion, typically the benefit agent-containing cleaning system of the present invention should be topically applied to affected body parts at regular intervals, and preferably from about 2 to about 14 times per week. More preferably, the composition/emulsion is applied more frequently during the initial stages of treatment, e.g. from about 5 to about 7 times per week until the desired effect is achieved, then less frequently when maintenance is desired, e.g. from about 2 to about 5 times per week.

The above-described skin conditioning composition is capable of efficiently mediating the deposition and permeation of various benefit agents, such as antidandruff agents, onto and into the skin following topical administration thereto.

Another preferred embodiment of the present invention is directed to a method for treating acne and for reducing the signs of aging, i.e. wrinkles, fine lines, and other manifestations of photodamage, comprising topically applying the above-described cleaning system and the relevant benefit agent to the skin of an animal or human at a desired area, wherein the benefit agent is comprised of an effective amount of an anti-acne agent or an anti-aging agent, respectively.

Examples of suitable anti-aging agents include, but are not limited to inorganic sunscreens such as titanium dioxide and zinc oxide; organic sunscreens such as octyl-methoxy cinnamates and derivatives thereof; retinoids; vitamins such as vitamin E, vitamin A, vitamin C, vitamin B, and derivatives thereof such as vitamin E acetate, vitamin C palmitate, and the like; antioxidants including beta carotene, alpha hydroxy acids such as glycolic acid, citric acid, lactic acid, malic acid, mandelic acid, ascorbic acid, alpha-hydroxybutyric acid, alpha-hydroxyisobutyric acid, alpha-hydroxyisocaprolc acid, atrolactic acid, alpha-hydroxyisovaleric acid, ethyl pyruvate, galacturonic acid, glucoheptonic acid, glucoheptono 1,4-lactone, gluconic acid, gluconolactone, glucuronic acid, glucuronolactone, glycolic acid, isopropyl pyruvate, methyl pyruvate, mucic acid, pyruvic acid, saccharic acid, saccharic acid 1,4-lactone, tartaric acid, and tartronic acid; beta hydroxy acids such as beta-hydroxybutyric acid, beta-phenyl-lactic acid, beta-phenylpyruvic acid; botanical extracts such as green tea, soy, milk thistle, algae, aloe, angelica, bitter orange, coffee, goldthread, grapefruit, hoellen, honeysuckle, Job's tears, lithospermum, mulberry, peony, puerarua, nice, safflower, and mixtures thereof.

Preferred anti-aging agents include retinoids, anti-oxidants, alpha-hydroxy acids and beta-hydroxy acid with retinol and tretinoin being most preferred. Suitable amounts of anti-aging agents include, based upon the total weight of the described cleaning system, from about 0.01 percent to about 20 percent, and preferably from about 0.04 percent to about 5 percent.

Examples of suitable anti-acne agents include, but are not limited to topical retinoids (tretinoin, isotretinoin, motretinide, adapalene, tazarotene, azelaic acid, retinol); salicylic acid; benzoyl peroxide; resorcinol; antibiotics such as tetracycline and isomers thereof, erythromycin, and the anti-inflammatory agents such as ibuprofen, naproxen, hetprofen; botanical extracts such as alnus, arnica, artemisia capillaris, asiasarum root, birrh, calendula, chamomile, cnidium, comfrey, fennel, galla rhois, hawthorn, houttuynia, hypericum, jujube, kiwi, licorice, magnolia, olive, peppermint, philodendron, salvia, sasa albo-marginata; imidazoles such as ketoconazole and elubiol, and those described in Gollnick, H et al. 196(1) Dermatology Sebaceous Glands, Acne and Related Disorders, 119-157 (1998), and mixtures thereof.

Preferred anti-acne agents include benzoyl peroxide, retinol, elubiol, antibiotics, and salicylic acid, with retinol and tretinoin being most preferred.

Suitable amount of anti-acne agents include, based upon the total weight of the described cleaning system, from about 0.01 percent to about 10 percent, and preferably from about 0.04 percent to about 5 percent.

Another preferred embodiment of the present invention is directed to a method for depigmenting the skin, comprising topically applying to skin at a desired area the above-described cleaning system and an effective amount of the depigmentation benefit agent. Suitable effective amounts of depigmentation agents include, based upon the total weight of the described cleaning system, from about 0.01 percent to about 10 percent, and preferably from about 0.04 percent to about 5 percent.

Examples of suitable depigmentation agents include, but are not limited to soy and derivatives thereof, retinoids such as retinol; Kojic acid and its derivatives such as, for example, kojic dipalmitate; hydroquinone and it derivatives such as arbutin; transexamic acid; vitamins such as niacin, vitamin C and its derivatives; azelaic acid; placertia; licorice; extracts such as chamomile and green tea, and mixtures thereof, with retinol, Kojic acid, and hydroquinone, being preferred.

An alternative preferred embodiment of the present invention is directed to a method for treating the symptoms and/or the diseases of dandruff, seborrheic dermatitis and/or psoriasis, comprising topically applying the above-described cleaning system and the relevant benefit agent to a location desired wherein the benefit agent is comprised of an effective amount of a dandruff treatment agent, a seborrheic dermatitis treatment agent, or a psoriasis treatment agent, respectively. As used herein, "dandruff treatment agent," "seborrheic dermatitis treatment agent," or a "psoriasis treatment agent," respectively, shall include agents capable of treating the symptoms and/or the diseases of dandruff, seborrheic dermatitis, and psoriasis, respectively. By "effective amount," it is meant an amount effective for treating the disease and/or the symptoms associated therewith and preferably may range from, based upon the total weight of the cleaning system, from about 0.001 percent to about 10 percent, and preferably from about 0.01 percent to about 5 percent.

Examples of benefit agents suitable for treating the symptoms and/or the diseases of dandruff, seborrheic dermatitis and/or psoriasis, respectively, nonexclusively include those set forth above with shale oil and derivatives thereof, elubiol, ketoconazole, coal tar, salicylic acid, zinc pyrithione, selenium sulfide, hydrocortisone, sulfur, menthol, pramoxine hydrochloride, and mixtures thereof being particularly preferred.

The compositions of the present invention may be directed applied to the skin or may be applied onto other delivery implements such as wipes, sponges, brushes, and the like. The compositions may be used in products designed to be left on the skin, wiped from the skin, or rinsed off of the skin.

The invention illustratively disclosed herein suitably may be practiced in the absence of any component, ingredient, or step which is not specifically disclosed herein. Several examples are set forth below to further illustrate the nature of the invention and the manner of carrying it out. However, the invention should not be considered as being limited to the details thereof.

### Vehicles /Carriers

In embodiments of the present invention related to skin care compositions, the compositions of the present invention comprise a safe and effective amount of a dermatologically-acceptable carrier, suitable for topical application to the skin within which the essential materials and optional other materials are incorporated to enable the essential materials and optional components to be delivered to the skin at an appropriate concentration. The carrier can thus act as a diluent, dispersant, solvent, or the like for the active ingredients of the composition. The carrier ensures that the active ingredients of the composition can be applied to and distributed evenly over the selected target at an appropriate concentration.

The carrier can be solid, semi-solid or liquid. The carriers may be liquid or semi-solid, such as creams, lotions and gels. The carrier can itself be inert or it can possess dermatological benefits of its own. The carrier should also be physically and, chemically compatible with the essential components described herein, and should not unduly impair stability, efficacy or other use benefits associated with the compositions of the present invention.

The type of carrier utilized in the present invention depends on the type of product form desired for the composition. The topical compositions useful in the subject invention may be made into a wide variety of product forms such as are known in the art. These include, but are not limited to, lotions, creams, gels, sticks, sprays, ointments, pastes, and mousses. These product forms may comprise several types of carriers including, but not limited to, solutions, aerosols, emulsions, gels, solids, and liposomes. The compounds which are active in the compositions and methods of this invention may be delivered topically be any means known to those of skill in the art. If the delivery parameters of the topically active pharmaceutical or cosmetic agent so require, the topically active composition may be further composed of a pharmaceutically or cosmetically acceptable vehicle capable of functioning as a delivery system to enable the penetration of the topically active agent into the skin.

The composition described herein can be prepared in the form of a cosmetic composition. The composition can be prepared in the form of basic cosmetic compositions (facial cleansers, such as toilet water, cream, essence, cleansing foam and cleansing water, pack and body oil), color cosmetic compositions (foundation, lipstick, mascara, and make-up base), hair product compositions (shampoo, rinse, hair conditioner and hair gel) and soap etc., which comprise the composition described herein as an active ingredient, together with a dermatologically acceptable carrier. The cosmetic composition can be easily prepared in any method known in the art, using the composition described herein together with at least one carrier and additives, which are commonly used in the field of preparing cosmetic compositions. Examples of cosmetic agents include emollients, humectants, colorants, pigments, fragrances, moisturizers, viscosity modifiers and any other cosmetic forming agent. One or more cosmetic agents can be included in the cosmetic composition. The form of the cosmetic composition can be a powder, lotion, gel, spray, stick cream, ointment, liquid, emulsion, foam or aerosol. In another embodiment, additional active ingredients as known in the art and described herein may also be used. Examples of the carriers may include, but are not limited to, a skin softener, a skin permeation enhancer, a colorant, an aromatic, an emulsifier, a thickener, and a solvent. Also, the cosmetic composition may further comprise a perfumery, a pigment, a bactericidal agent, an antioxidant, a preservative and a moisturizer, and also a thickener, inorganic salts and synthetic polymer substances, for the purpose of improving physical properties. Lists of such materials, and formulations for the creation of particular types of lotions, creams, sunscreens, lipsticks and other such forms are widely available in the patent literature and in commercial handbooks and can be used by those skilled in the preparation of such formulations to incorporate the composition described herein.

In one example, the facial cleanser and soap, which comprise the composition described herein, can be easily prepared by adding composition to the facial cleanser base and soap base. The cream can be prepared by adding the composition to a general oil-in-water (0/W) cream base. The cleanser, soap and cream may farther comprise a perfumery, a chelating agent, a pigment, an antioxidant and a preservative, and also synthetic or natural materials, proteins, minerals and vitamins, for the purpose of improving physical properties. Detailed descriptions of methods by which the composition may be delivered are described below. However, is should be understood that these are not limited descriptions, but merely used to illustrate possible embodiments of the invention.

### Topical Delivery via Liposomes

One acceptable vehicle for topical delivery of some of the compositions of this invention, particularly those which include proteins, may contain liposomes. The liposomes may be present in an amount, based upon the total volume of the composition, from about 10 mg/mL to about 100 mg/mL, or from about 20 mg/mL to about 50 mg/mL. The liposome may have a ratio of about 37.5:12.5:33.3:16.7. Suitable liposomes may be prepared in accordance with the protocol set forth in application U.S. Serial No. 09/110,409, though other methods commonly used in the art are also acceptable. The above described composition may be prepared by combining the desired components in a suitable container and mixing them under ambient conditions in any conventional high shear mixing means well known in the art for non-ionic liposomes preparations, such as those disclosed in Niemiec et al., "Influence of Nonionic Liposomal Composition On Topical Delivery of Peptide Drugs Into Pilosebacious Units: An In Vivo Study Using the Hamster Ear Model," 12 Pharm. Res. 1184-88 (1995) ("Niemiec"). We have found that the presence of these liposomes in the compositions of this invention may enhance the therapeutic capabilities of some of the compositions of this invention.

### Topical Delivery via a Solution

The topical compositions useful in the present invention can be formulated as solutions. Solutions typically include an aqueous solvent (e.g., from about 50% to about 99.99% or from about 90% to about 99% of a cosmetically acceptable aqueous solvent).

### Topical Delivery via Lotions, Creams, Emollients, or Ointments

The composition may also be delivered topically via a lotion. Lotions typically comprise from about 1% to about 20% (e.g., from about 5% to about 10%) of an emollient(s) and from about 50% to about 90% (e.g., from about. 60% to about 80%) of water. Another type of product that may be formulated from a solution is a cream. A cream typically comprises from about 5% to about 50% (e.g., from about 10% to about 20%) of an emollient(s) and from about 45% to about 85% (e.g., from about 50% to about 75%) of water. Single emulsion skin care preparations, such as lotions and creams, of the oil-in-water type and water-in-oil type are well-known in the cosmetic art and are useful in the subject invention. Multiphase emulsion compositions, such as the water-in-oil-in- water type are also useful in the subject invention. In general, such single or multiphase emulsions contain water, emollients, and emulsifiers as essential ingredients.

Creams can be made with a support that is a soap-based or fatty alcohol-based formula in the presence of an emulsifier. The soaps can be any known in the cosmetic formulation art, and include natural fatty or synthetic acids having from 12 to 20 carbon atoms (such as lauric acid, myristic acid, palmitic acid, oleic acid, stearic acid and their mixtures) in concentrations of from 10 to 30% neutralized with cosmetically acceptable salts including sodium, potassium, ammonia, monoethanolamine, triethanolamine and their mixtures.

Topical compositions useful in the subject invention may be formulated as a solution comprising an emollient. Such compositions may contain from about 2% to about 50% of an emollient(s). As used herein, "emollients" refer to materials used for the prevention or relief of dryness, as well as for the protection of the skin. When the conditioning agent is an emollient it may be selected from hydrocarbons, fatty acids, fatty alcohols and esters. Isononyl isononanoate is a hydrocarbon type of emollient conditioning agent. Other hydrocarbons that may be employed include mineral oil, polyolefins such as polydecene, and paraffins such as isohexadecane (e.g. Permethyl 99® and Permethyl 101®). Other examples of such materials are described in WO 96/16636, incorporated by reference herein. One material that may be used is known by the INCI name sucrose polycottonseedate.

Organopolysiloxane oils: The organopolysiloxane oil may be volatile, non-volatile, or a mixture of volatile and non-volatile silicones. The term "non-volatile" as used in this context refers to those silicones that are liquid under ambient conditions and have a flash point (under one atmospheric of pressure) of or greater than about 100° C. The term "volatile" as used in this context refers to all other silicone oils. Suitable organopolysiloxanes can be selected from a wide variety of silicones spanning a broad range of volatilities and viscosities. Non-volatile polysiloxanes are preferred. Suitable silicones are disclosed in U.S. Pat. No. 5,069,897, issued Dec. 3. Organopolysiloxanes selected from the group consisting of polyalkylsiloxanes, alkyl substituted dimethicones, dimethiconols, polyalkylaryl siloxanes, and mixtures thereof may be used. Polyalkylsiloxanes, dimethicones and cyclomethicones may be used.

Vegetable oils and hydrogenated vegetable oils: Examples of vegetable oils and hydrogenated vegetable oils include safflower oil, castor oil, coconut oil, cottonseed oil, menhaden oil, palm kernel oil, palm oil, peanut oil, soybean oil, rapeseed oil, linseed oil, rice bran oil, pine oil, sesame oil, sunflower seed oil, partially and fully hydrogenated oils from the foregoing sources, and mixtures thereof. Animal fats and oils, e.g. cod liver oil, lanolin and derivatives thereof such as acetylated lanolin and isopropyl lanolate may be used. Also useful are C₄-C₂₀ alkyl ethers of polypropylene glycols, C₁-C₂₀ carboxylic acid esters of polypropylene glycols, and di-C₈-C₃₀ alkyl ethers, examples of which include PPG-14 butyl ether, PPG-15 stearyl ether, dioctyl ether, dodecyl octyl ether, and mixtures thereof. The compositions of the present invention may be substantially free of semi-solid hydrocarbons such as petrolatum, lanolin and lanolin derivatives, sterols (e.g., ethoxylated soya sterols), high molecular weight polybutenes and cocoa buffer. By "substantially free," as used herein, means that the concentration of the semi-solid hydrocarbons are less than 10%, or less than 5% or less than 2% or 0.

C₁-C₃₀ mono- and poly-esters of sugars and related materials: These esters are derived from a sugar or polyol moiety and one or more carboxylic acid moieties. Depending on the constituent acid and sugar, these esters can be in either liquid or solid form at room temperature. Examples of liquid esters include: glucose tetraoleate, the glucose tetraesters of soybean oil fatty acids (unsaturated), the marmose tetraesters of mixed soybean oil fatty acids, the galactose tetraesters of oleic acid, the arabinose tetraesters of linoleic acid, xylose tetralinoleate, galactose pentaoleate, sorbitol tetraoleate, the sorbitol hexaesters of unsaturated soybean oil fatty acids, xylitol pentaoleate, sucrose tetraoleate, sucrose pentaoletate, sucrose hexaoleate, sucrose hepatoleate, sucrose octaoleate, and mixtures thereof. Examples of solid esters include: sorbitol hexaester in which the carboxylic acid ester moieties are palmitoleate and arachidate in a **1:2** molar ratio; the octaester of raffinose in which the carboxylic acid ester moieties are linoleate and behenate in a 1:3 molar ratio; the heptaester of maltose wherein the esterifying carboxylic acid moieties are sunflower seed oil fatty acids and lignocerate in a 3:4 molar ratio; the octaester of sucrose wherein the esterifying carboxylic acid moieties are oleate and behenate in a 1:3 molar ratio; and the octaester of sucrose wherein the esterifying carboxylic acid moieties are laurate, linoleate and behenate in a 1:3:4 molar ratio. A solid material that may be used is sucrose polyester in which the degree of esterification is 7-8, and in which the fatty acid moieties are C18 mono- and/or di-unsaturated and behenic, in a molar ratio of unsaturates:behenic of 1:7 to 3:5. A solid sugar polyester that may be used is the octaester of sucrose in which there are about 7 behenic fatty acid moieties and about 1 oleic acid moiety in the molecule. Other materials include cottonseed oil or soybean oil fatty acid esters of sucrose. The ester materials are further described in, U.S. Pat. No. 2,831,854, U.S. Pat. No. 4,005,196, to Jandacek, issued Jan. 25, 1977; U.S. Pat. No. 4,005,195, to Jandacek, issued Jan. 25, 1977, U.S. Pat. No. 5,306,516, to Letton et al., issued Apr. 26, 1994; U.S. Pat. No. 5,306,515, to Letton et al., issued Apr. 26, 1994; U.S. Pat. No. 5,305,514, to Letton et al., issued Apr. 26, 1994; U.S. Pat. No. 4,797,300, to Jandacek et al., issued Jan. 10, 1989; U.S. Pat. No. 3,963,699, to Rizzi et al, issued Jun. 15, 1976; U.S. Pat. No. 4,518,772, to Volpenhein, issued May 21, 1985; and U.S. Pat. No. 4,517,360, to Volpenhein, issued May 21, 1985.

The topical compositions of the subject invention generally comprise from about 1% to about 50%, or about 3% to about 15% of a dermatologically acceptable emollient. Emollients tend to lubricate the skin, increase the smoothness and suppleness of the skin, prevent or relieve dryness of the skin, and/or protect the skin. Emollients are typically water-immiscible, oily or waxy materials. A wide variety of suitable emollients are known and may be used herein. Sagarin, Cosmetics, Science and Technology, 2nd Edition, Vol. 1, pp. 32-43 (1972), contains numerous examples of materials suitable as an emollient; See also International Cosmetic Ingredient Dictionary and Handbook, eds. Wenninger and McEwen, pp. 1656-61, 1626, and 1654-55 (The Cosmetic, Toiletry, and Fragrance Assoc., Washington, D.C., 7 (th) Edition, 1997) (hereinafter "INCI Handbook") contains numerous examples of suitable materials. Emollients include stearyl alcohol, glyceryl monostearate, propane-1,2-diol, butane-1,3-diol, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, cetyl palmitate, polydimethylsiloxane, di-n-butyl sebacate, isopropyl myristate, isopropyl palmitate, isopropyl stearate, butyl stearate, polyethylene glycol, triethylene glycol, lanolin, sesame oil, coconut oil, arachis oil, acetylated lanolin alcohols, petroleum, mineral oil, butyl myristate, isostearic acid, palmitic acid, isopropyl linoleate, lauryl lactate, myristyl lactate, decyl oleate, myristyl myristate.

Yet another type of product that may be formulated from a solution is an ointment. An ointment may comprise a simple base of animal or vegetable oils or semi-solid hydrocarbons. An ointment may comprise from about 2% to about 10% of an emollient(s) plus from about 0.1% to about 2% of a thickening agent(s). Examples of thickening agents include cellulose derivatives (methyl cellulose and hydroxyl propylmethylcellulose), synthetic high molecular weight polymers (e.g., carboxyvinyl polymer and polyvinyl alcohol), plant hydrocolloids (e.g., karaya gum and tragacanth gum), clay thickeners (e.g., colloidal magnesium aluminium silicate and bentonite), and carboxyvinyl polymers (CARBOPOLS; sold by B.F. Goodrich Company, (such polymers are described in Brown, US Pat. NO. 2,798,053), carboxylic acid polymers, crosslinked polyacrylates, polyacrylamides, xanthan gum and mixtures thereof. See also Sagarin, Cosmetics, Science and Technology , 2nd Ed. Vol.1 or INCI Handbook pp. 1693-1697, for a more complete disclosure of thickening agents or viscosity increasing agents useful herein.

### Topical Delivery via an Emulsion

The topical compositions useful in the present invention may be formulated as emulsions. If the carrier is an emulsion, from about 1% to about 10% (e.g., from about 2% to about 5%) of the carrier comprises an emulsifier(s). Emulsifiers may be nonionic, anionic or cationic. Suitable emulsifiers are disclosed in, for example, INCI Handbook, pp. 1673-1686. Lotions and creams can be formulated as emulsions. Typically such lotions comprise from 0.5% to about 5% of an emulsifier(s). Such creams would typically comprise from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

### Topical Delivery via a Gel

The topical compositions of this invention can also be formulated as a gel (e.g., an aqueous gel using a suitable gelling agent(s)). Suitable gelling agents for aqueous gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically comprise between about 0.1% and 5%, by weight, of such gelling agents.

### Topical Delivery via a Solid Formulation

The topical compositions of the present invention can also be formulated into a solid formulation (e.g., a wax-based stick, soap bar composition, powder, or a wipe containing liquid or powder).

The topical compositions useful in the subject invention may contain, in addition to the aforementioned components, a wide variety of additional oil-soluble materials and/or water-soluble materials conventionally used in compositions for use on skin, hair, and rails at their art-established levels.

### Liquid vehicles

Compositions can include at least one cosmetically acceptable vehicle other than water. Vehicles other than water include solids or liquids such as emollients, solvents, humectants, thickeners and powders. Suitable liquid vehicles include mineral oil, silicone oil, lipids (such as lecithin, vegetable oil, vitamin E, and derivatives of lanolin), low-molecular weight glycols such as PEG-4, PEG-6, propylene glycol, glycerin, and their lower alkyl conjugates, and ketones, such as acetone. Solvents containing unconjugated hydroxyls may undergo slow exchange with the ester groups of the polyhydroxy acids; this may affect the bulk consistency of the preparation, but would still result in slow release of the hydroxy acid monomers. Solvents include ethyl alcohol, isopropanol, ethylene glycol monoethyl ether, diethylene glycol monobutyl ether, diethylene glycol monoethyl ether, and acetone.

### Additional Components

Nonlimiting examples of these additional ingredients include additional skin care actives such as peptides (e.g., Matrixyl [pentapetide derivative]), farnesol, bisabolol, phytantriol, glycerol, urea, guanidine (e.g., amino guanidine); vitamins and derivatives thereof such ascorbic acid, vitamin A (e.g., retinoid derivatives such as retinyl palmitate or retinyl proprionate), vitamin E (e.g., tocopherol acetate), vitamin B3 (e.g., niacinamide) and vitamin B5 (e.g., panthenol) and the like and mixtures thereof; sunscreens; anti-acne medicaments (resorcinol, salicylic acid, and the like; antioxidants (e.g., phytosterols, lipoic acid); flavonoids (e.g., isoflavones, phytoestrogens); skin soothing and healing agents such as aloe vera extract, allantoin and the like; chelators and sequestrants; and agents suitable for aesthetic purposes such as essential oils, fragrances, skin sensates, opacifiers, aromatic compounds (e.g., dove oil, menthol, camphor, eucalyptus oil, and eugenol). Nonlimiting examples of suitable carboxylic copolymers, emulsifiers, emollients, and other additional ingredients are disclosed in U.S. Pat. No. 5,011,681, to Ciotti et al., issued Apr. 30, 1991 and U.S. Pat. No. 5,939,082, to Oblong et al., issued Aug. 17, 1999. The above-mentioned vitamin B3 compounds can be incorporated as re-crystallized crystals that remain in crystalized form in the composition or as partially solubilize crystals (i.e. some of the crystals are dissolved and some remain in crystalline form in the composition.).

Various other agents may be incorporated into the composition depending on therapeutic need, desired results or for purposes of shelf-life and stability.

### Film-Forming Agents

Film-forming agents may be optionally included in the compositions of the present invention to aid film substantivity and adhesion to the skin. Improving the long wear and non-transfer performance of the present compositions is quite desirable. Water-soluble, water insoluble, and water dispersible film forming agents can be used in the internal and external phases of the present compositions to give the desired end benefit.

The compositions may comprise from about 0% to about 20%, or from about 0.1% to about 10%, or from about 0.1% to about 5%, by weight of the composition, of the film-forming agent.

Film forming agents that may be used with the present invention include: 1) organic silicone resins, fluorinated silicone resins, copolymers of organic silicone resins, e.g., trimethylsiloxysilicate from GE (SR1000), GE's copolymers of silicone resins, e.g., SF1318 (silicone resin and an organic ester of isostearic acid copolymer) and CF1301 (silicone resin and alpha methyl styrene copolymer), Dow Corning's pressure sensitive adhesives--copolymers of silicone resins and various PDMS's (BIO-PSA series); and 2) acrylic and methacrylic polymers and resins, silicone-acrylate type copolymers and fluorinated versions of, including--silicones plus polymer SA70 from 3M, KP545 from Shin-Etsu, alkyl-acrylate copolymers, e.g., IEP 561 and 562 from Shin-Etsu; 3) decene/butene copolymer from Collaborative Labs; 4) polyvinyl based materials, e.g., PVP, PVP/VA, including Antaron/Ganex from ISP (PVP/Triacontene copolymer), Luviskol materials from BASF; 5) polyurethanes, e.g., the Polyderm series from Alzo including but not limited to Polyderm PE/PA, Polyderm PPI-SI-WS, Polyderm PPI-GH, Luviset P.U.R. from BASF; 6) polyquaternium materials, e.g., Luviquat series from BASF 7) acrylates copolymers and acrylates/acrylamide copolymers, e.g., Luvimer and Ultrahold series, both available from BASF; 8) styrene-based materials; and 9) chitosan and chitosan-based materials including cellulose and cellulose-based materials. Such film formers are disclosed for example in the International Cosmetic Ingredient Dictionary and Handbook, Seventh Edition, Vol 2, 1636-1638.

### Thickening Agents

The compositions of the present invention may optionally also comprise a thickening agent from about 0.1% to about 5%, or from about 0.1% to about 3%, or from about 0.25% to about 2%, of a thickening agent.

Thickening agents that may be used with the present invention include cellulose and derivatives such as cellulose, carboxymethyl hydroxyethylcellulose, cellulose acetate propionate carboxylate, hydroxyethylcellulose, hydroxyethyl ethylcellulose, hydroxypropylcellulose, hydroxypropyl methylcellulose, methyl hydroxyethylcellulose, microcrystalline cellulose, sodium cellulose sulfate, and mixtures thereof. Also useful herein are the alkyl substituted celluloses. Examples of alkyl groups useful herein include those selected from the group consisting of stearyl, isostearyl, lauryl, myristyl, cetyl, isocetyl, cocoyl (i.e. alkyl groups derived from the alcohols of coconut oil), palmityl, oleyl, linoleyl, linolenyl, ricinoleyl, behenyl, and mixtures thereof. The material sold under the tradename Natrosol™. CS Plus from Aqualon Corporation may be used.

Other useful thickeners include acacia, agar, algin, alginic acid, ammonium alginate, amylopectin, calcium alginate, calcium carrageenan, carnitine, carrageenan, dextrin, gelatin, gellan gum, guar gum, guar hydroxypropyltrimonium chloride, hectorite, hyaluroinic acid, hydrated silica, hydroxypropyl chitosan, hydroxypropyl guar, karaya gum, kelp, locust bean gum, natto gum, potassium alginate, potassium carrageenan, propylene glycol alginate, sclerotium gum, sodium carboxymethyl dextran, sodium carrageenan, tragacanth gum, xanthan gum, and mixtures thereof. Also useful are acrylic acid/ethyl acrylate copolymers and the carboxyvinyl polymers sold by the B.F. Goodrich Company under the trade mark of Carbopol resins. Suitable Carbopol resins are described in WO98/22085.

### Shine-Control Agents

Cosmetic products that improve and/or regulate the condition of the shiny appearance of skin are increasingly popular with consumers and are referred to herein as "shine-control agents." Shine control agents may be included in the compositions of the present invention.

A frequent, undesirable condition is "oily skin", which results from the excessive amount of sebum and sweat that is excreted onto the skin. Sebum is an oily mixture, composed principally of squalene, triglycerides, fatty acids and wax esters. Sebum is produced in the sebaceous glands of the skin. Oily skin is associated with a shiny, undesirable appearance and disagreeable tactile sensation. Sweat is predominantly water with trace quantities of dissolved inorganic salts such as sodium chloride and potassium chloride.

Typically, shine-control agents are porous in nature. These agents, when applied to the skin provide a reservoir to absorb excess moisture into the pores, hence reducing the visible quantity of moisture on the skin.

Absorber and non-absorbing spherical particles may be combined to provide optimum shine control as well as providing a product with the best tactile sensory performance.

Suitable shine-control agents include, but are not limited to, silicas, magnesium aluminium silicates, talc, sericite and various organic copolymers. Particularly effective shine control agents include silicates or carbonates that are formed by reaction of a carbonate or silicate with the alkali (IA) metals, alkaline earth (IIA) metals, or transition metals, and silicas (silicon dioxide). Shine control agents that may be used include calcium silicates, amorphous silicas, calcium carbonates, magnesium carbonates, zinc carbonates, and combinations thereof. Some specific examples of the silicates and carbonates useful in this present invention are more fully explained in Van Nostrand Reinhold's Encyclopedia of Chemistry, 4th Ed. pp155, 169, 556, and 849 (1984).

Synthetic versions of the shine-control agents, particularly silicates, may be used. Suitable synthetic carbonates are commercially available from Mallinckrodt or Whittaker, Clarke & Daniels. Examples of synthetic silicates useful in the present invention are Hubersorb 250™ or Hubersorb 600™., available from J.M. Huber.

Shine-control agents that primarily comprise silicas may be used instead of those materials comprising mainly silicates and/or carbonates when used for moisture and shine control. Silicas may also be in the form of microspheres and/or ellipsoids, as they have been found to contribute good skin feel characteristics in addition to efficient moisture absorption. Silica ellipsoids useful in the present invention are available from DuPont as ZELEC Sil and Kobo as Silica Shells. Silica microspheres are available from Kobo as MSS-500, MSS500/3, MSS-500H, MSS500/3N, MSS-500N and MSS 500/3N; Presperse as Spheron L1500, Spheron P1500. Fumed versions of silica can also be used with Aerosil from Degussa and Cab-O-Sil from Cabot both being useful.

Amongst the silicate series, magnesium aluminium silicates such as Sebumase, available from Miyoshi Kasei are useful.

When silicas, such as silica ellipsoids and silica microspheres are intended to be the main means for moisture absorption, the absorbent powder may comprise from about 1% to about 40%; or about 1% to about 25%, or about 2% to about 10%, by weight of the composition, of silicas.

Starch-based materials may also be used as shine control agents. Examples include Natrosorb W and Natrosorb HFW, DryFlo plus and DryFlo AF pure from National Starch and Chemical Company.

### Skin-Conditioning Agents

Optionally, the compositions of the present invention may further comprise a skin-conditioning agent. These agents may be selected from humectants, exfoliants or emollients. Amounts of the skin-conditioning agent may range from about 0% to about 30%, or from about 1% to about 20%, or from about 1% to about 10% by weight.

### Humectants

Humectants are generally substances that can attract water, usually out of the air. Humectants are generally considered to be moisturizers. Suitable humectants that can be used with the present invention include but are not limited to glycerin, sorbitol, sodium 2-pyrrolidone-5-carboxylate, soluble collagen, dibutyl phthalate, gelatin, and poly(alkylene oxide)s, such as polyethylene glycol. Sodium carbonate is used in the art as a humectant.

### Desquamation Agents/Exfoliants

A safe and effective amount of a desquamation agent may be added to the compositions of the subject invention, or from about 0.1% to about 10%, or from about 0.2% to about 5%, or from about 0.5% to about 4% of the composition. Desquamation agents enhance the skin appearance benefits of the present invention. For example, the desquamation agents tend to improve the texture of the skin (e.g., smoothness). A variety of desquamation agents are known in the art and are suitable for use herein, including organic hydroxy acids such as salicylic acid, glycolic acid, lactic acid, 5-octanoyl salicylic acid, hydroxyoctanoic acid, hydroxycaprylic acid, and lanolin fatty acids. One desquamation system that may be used comprises sulphydryl compounds and zwitterionic surfactants and is described in WO 96/01101. Another desquamation system that may be used comprises salicylic acid and zwitterionic surfactants and is described in WO 95/13 048.

Exfoliants that may be used with the present invention include but are not limited to C2-C30 alpha-hydroxycarboxylic acids, beta-hydroxycarboxylic acids and salts of these acids. Glycolic, lactic and salicylic acids and their ammonium salts may be used. Amounts of the exfoliants may range from about 1 to about 15%, or from 2 to 10% by weight.

A wide variety of C2-C30 alpha-hydroxycarboxylic acids may be employed. Suitable examples of which include: alpha-hydroxyethanoic acid, alpha-hydroxypropanoic acid, alpha-hydroxyhexanoic acid, alpha-hydroxyoctanoic acid, alpha-hydroxydecanoic acid, alpha-hydroxydodecanoic acid, alpha-hydroxytetradecanoic acid, alpha-hydroxyhexadecanoic acid, alpha-hydroxyoctadecanoic acid, alpha-hydroxyeicosanoic acid, alphahydroxydocosanoic acid, alpha-hydroxyhexacosanoic acid, and alphahydroxyoctacosanoic acid.

### Powders

Powders useful alone in dry compositions or as fillers in liquid compositions include chalk, talc, fullers earth, kaolin, starch, guurs, colloidal silicon dioxide, sodium polyacrylate, tetra alkyl and/or trialkyl aryl ammonium silicate, organically modified montmorillonite day, hydrated aluminium silicate, fumed silica, carboxyvinyl polymer, and sodium carboxymethyl cellulose.

### Gels

Cosmetic gels may contain thickening or gelling agents such as sodium alginate or arabic gum or cellulose derivatives optionally in the presence of a solvent. The thickening agent concentration may range from about 0.5 to about 30 weight percent or from about 0.5 to about 15 weight percent. Solvents used can be aliphatic lower alcohols, glycols and their ethers with the concentration of the solvents ranging from about 2 to about 20%.

### Solidifying Agents

The cosmetic compositions of this invention may also contain one or more materials, herein singly or collectively referred to as a "solidifying agent," that are effective to solidify the particular liquid base materials to be used in a cosmetic composition. (As used herein, the term "solidify" refers to the physical and/or chemical alteration of the liquid base material so as to form a solid or semi-solid at ambient conditions, i.e., to form a final composition that has a stable physical structure and is deposited on the skin during normal use conditions.) As is appreciated by those skilled in the art, the selection of the particular solidifying agent for use in the cosmetic compositions will depend upon the particular type of composition desired, i.e., gel or wax-based, the desired rheology, the liquid base material used and the other materials to be used in the composition. The solidifying agent may be present at a concentration of from about 0 to about 90%, or from about 1 to about 50%, or from about 5% to about 40%, or from about 1% to about 15%.

Suitable solidifying agents may include waxy materials such as candelilla, carnauba, beeswax, spermaceti, carnauba, baysberry, montan, ozokerite, ceresin, paraffin, synthetic waxes such as Fisher-Tropsch waxes, silicone waxes (e.g., DC 2503 from Dow Corning), microcrystalline waxes and the like; soaps, such as the sodium and potassium salts of higher fatty acids, i.e., acids having from 12 to 22 carbon atoms; amides of higher fatty acids; higher fatty acid amides of alkylolamines; dibenzaldehyde-monosorbitol acetals; alkali metal and alkaline earth metal salts of the acetates, propionates and lactates; and mixtures thereof. Also useful are polymeric materials such as, locust bean gum, sodium alginate, sodium caseinate, egg albumin, gelatin agar, carrageenin gum sodium alginate, xanthan gum, quince seed extract, tragacanth gum, starch, chemically modified starches and the like, semisynthetic polymeric materials such as cellulose ethers (e.g. hydroxyethyl cellulose, methyl cellulose, hydroxypropyl cellulose, carboxymethyl cellulose, hydroxy propylmethyl cellulose), polyvinylpyrrolidone, polyvinylalcohol, guar gum, hydroxypropyl guar gum, soluble starch, cationic celluloses, cationic guars and the like and synthetic polymeric materials such as carboxyvinyl polymers, polyvinylpyrrolidone, polyvinyl alcohol polyacrylic acid polymers, polymethacrylic acid polymers, polyvinyl acetate polymers, polyvinyl chloride polymers, polyvinylidene chloride polymers and the like. Inorganic thickeners may also be used such as aluminium silicates, such as, for example, bentonites, or a mixture of polyethylene glycol and polyethylene glycol stearate or distearate. Naturally occurring polymers or biopolymers and their use are further described in European Application No. 522624, to Dunphy et al. Additional examples of naturally occurring polymers or biopolymers can be found in the Cosmetic Bench Reference, pp. 1.40-142.

### Spray Propellants

Spray compositions may require propellants, including propane, butane, isobutane, carbon dioxide, and nitrous oxide.

### Fragrances

The composition can also include a perfume in an amount sufficient to make the composition acceptable to the consumer and pleasant to use. Usually, the perfume will form from about 0.01% to about 10% by weight of the composition.

### Surfactants

Compositions herein may contain an emulsifier and/or surfactant, generally to help disperse and suspend the discontinuous phase within the continuous phase. A surfactant may also be useful if the product is intended for skin cleansing. For convenience hereinafter emulsifiers will be referred to under the term "surfactants", thus "surfactant(s)" will be used to refer to surface active agents whether used as emulsifiers or for other surfactant purposes such as skin cleansing. Known or conventional surfactants can be used in the composition, provided that the selected agent is chemically and physically compatible with essential components of the composition, and provides the desired characteristics. Suitable surfactants include silicone materials, non-silicone materials, and mixtures thereof.

The compositions of the present invention may comprise from about 0.05% to about 15% of a surfactant or mixture of surfactants. The exact surfactant or surfactant mixture chosen will depend upon the pH of the composition and the other components present. Surface active agents (detergents) useful in cosmetic compositions include anionic surface active agents, such as salts of fatty acids (for example, sodium laurate and triethanolamine oleate), alkyl benzene sulfonates (such as triethanolamine dodecyl benzene sulfonate), alkyl sulfates such as sodium lauryl sulfate, alkyl ether sulfates, monoglyceride sulfates, isethionates, methyl taurides, acylsarcosinates, acyl peptides, acyl lactylates, polyalkoxylated ether glycollates, for example trideceth-7 carboxylic acid, and phosphates such as sodium dilauryl phosphate. Amphoteric surface active agents include imidazole compounds, N-alkyl amino acids, (such as sodium cocaminopropionate and asparagine derivatives), and betaines. Nonionic surface active agents, such as fatty acid alkanolamides, for example, oleic ethanolamide; esters of polyalcohols, for example Span; polyglycerol esters; polyalkoxylated derivatives, for example TRITON X-100™, polyoxyethylene lauryl ether, and TWEEN™.; and amine oxides, such as dodecyl dienethyl amine oxides. Cationic surfactants may be of use in the composition. Mixtures of two or more of the above surface active agents can be employed in the composition. As noted above, many surfactants may also be used for their functionality as emollients, humectants or vehicles.

Other useful nonionic surfactants include the condensation products of alkylene oxides with fatty acids (i.e. alkylene oxide esters of fatty acids). These materials have the general formula RCO(X). OH wherein R is a C₁₀₋₃₀ alkyl group, X is --OCH₂CH₂-- (i.e. derived from ethylene glycol or oxide) or --OCH₂CHCH₃-- (i.e. derived from propylene glycol or oxide), and n is an integer from about 6 to about 200. Other nonionic surfactants are the condensation products of alkylene oxides with 2 moles of fatty acids (i.e. alkylene oxide diesters of fatty acids). These materials have the general formula RCO(X)ₙOOCR wherein R is a C₁₀₋₃₀ alkyl group, X is --OCH₂CH₂-(i.e. derived from ethylene glycol or oxide) or --OCH₂CHCH₃-- (i.e. derived from propylene glycol or oxide), and n is an integer from about 6 to about 100. Other nonionic surfactants are the condensation products of alkylene oxides with fatty alcohols (i.e. alkylene oxide ethers of fatty alcohols). These materials have the general formula R(X)ₙOR' wherein R is a C₁₀₋₃₀ alkyl group, X is --OCH₂CH₂-- (i.e. derived from ethylene glycol or oxide) or --OCH₂CHCH₃-- (i.e. derived from propylene glycol or oxide), and n is an integer from about 6 to about 100 and R' is H or a C₁₀₋₃₀ alkyl group. Still other nonionic surfactants are the condensation products of alkylene oxides with both fatty acids and fatty alcohols [i.e. wherein the polyalkylene oxide portion is esterified on one end with a fatty acid and etherified (i.e. connected via an ether linkage) on the other end with a fatty alcohol]. These materials have the general formula RCO(X)ₙOR' wherein R and R' are C₁₀₋₃₀ alkyl groups, X is -OCH₂CH₂ (i.e. derived from ethylene glycol or oxide) or --OCH₂CHCH₃ (derived from propylene glycol or oxide), and n is an integer from about 6 to about 100, examples of which include ceteth-6, ceteth-10, ceteth-12, ceteareth-6, ceteareth-10, ceteareth-12, steareth-6, steareth-10, steareth-12, PEG-6 stearate, PEG-10 stearate, PEG-100 stearate, PEG-12 stearate, PEG-20 glyceryl stearate, PEG-80 glyceryl tallowate, PEG-10 glyceryl stearate, PEG-30 glyceryl cocoate, PEG-80 glyceryl cocoate, PEG-200 glyceryl tallowate, PEG-8 dilaurate, PEG-10 distearate, and mixtures thereof.

Still other useful nonionic surfactants include polyhydroxy fatty acid amide surfactants, which are described in more detail in WO 98/04241.

Other nonionic surfactants suitable for use herein include sugar esters and polyesters, alkoxylated sugar esters and polyesters, C₁-C₃₀ fatty acid esters of C₁-C₃₀ fatty alcohols, alkoxylated derivatives of C₁-C₃₀ fatty acid esters of Ci-C₃₀ fatty alcohols, alkoxylated ethers of C₁-C₃₀ fatty alcohols, polyglyceryl esters of C₁-C₃₀ fatty acids, C₁-C₃₀ esters of polyols, C₁-C₃₀ ethers of polyols, alkyl phosphates, polyoxyalkylene fatty ether phosphates, fatty acid amides, acyl lactylates, and mixtures thereof. Examples of these non-silicon-containing surfactants include: polysorbate 20, polyethylene glycol 5 soya sterol, steareth-20, ceteareth-20, PPG-2 methyl glucose ether distearate, ceteth-10, polysorbate 80, polysorbate 60, glyceryl stearate, sorbitan monolaurate, polyoxyethylene 4 lauryl ether sodium stearate, polyglyceryl-4 isostearate, hexyl laurate, PPG-2 methyl glucose ether distearate, PEG-100 stearate, and mixtures thereof.

### Sunscreen

The composition may include an effective amount of a sunscreen agent to provide protection from the harmful effects of excessive exposure to sunlight. This can be particularly important when the skin is partially debrided by the application of a hydroxy acid. Examples of suitable organic sunscreens, when required, include Benzophenone-3, DEA Methoxycinnamate, Ethyl dihydroxypropyl PABA, Glyceryl PABA, Octyl methoxycinnamate, Octyl salicylate, 2-phenyl-benzimidazole-5-sulfonic acid, and Butyl methoxy dibenzoylmethane. An inorganic sunscreen, such as titanium dioxide or zinc oxide, can also be used. The sunscreen ingredients may be present free in the formulation. The organic sunscreens may also be coupled to the conjugate block copolymer, as described above.

### Anti-Oxidants

Anti-oxidants/radical scavengers such as ascorbic acid (vitamin C) and its salts, ascorbyl esters of fatty acids, ascorbic acid derivatives (e.g., magnesium ascorbyl phosphate), beta-carotene, tocopherol (vitamin E), tocopherol sorbate, tocopherol acetate, other esters of tocopherol, butylated hydroxy benzoic acids and their salts, gallic acid and its alkyl esters, such as propyl gallate, uric acid and its salts and alkyl esters, sorbic acid and its salts, amines (e.g., N,N-diethylhydroxylamine, amino-guanidine), sulfhydryl compounds (e.g., glutathione), dihydroxy fumaric acid and its salts, bioflavonoids, lysine, methionine, proline, superoxide dismutase, silymarin, tea extracts, grape skin/seed extracts, melanin, and rosemary extracts may be used. Anti-oxidants/radical scavengers that may be used include tocopherol acetate, tocopherol sorbate and other esters of tocopherol.

The tocopherol (vitamin E group) used as an additive or as an antioxidant for retinol, or a derivative, when present in the composition may include alpha-tocopherol, beta-tocopherol, gamma-tocopherol, and delta-tocopherol. The amount of tocopherol, when present in the composition, may be from about 0.0001 to about 50%, or from about 0.001 to about 10% by weight of the composition.

### Chelators

The composition may also further comprises biofilm dislodging enhancer agents such as chaotropic agents or calcium chelators.

The inclusion of a chelating agent is useful for providing protection against UV radiation which can contribute to excessive scaling or skin texture changes and against other environmental agents which can cause skin damage. A suitable amount is from about 0.01% to about 1%, or from about 0.05% to about 0.5%, of the composition. Exemplary chelators that are useful herein are disclosed in U.S. Pat. No. 5,487,884, incorporated herein by reference. Chelators that may be useful in compositions of the subject invention are ethylenediamine tetraacetic acid (EDTA), furildioxime and derivatives thereof.

A calcium chelator such as EDTA, may also be in a salt form, in a concentration of at least about 0.25%, or any calcium chelator having a chelating potency substantially equivalent thereto may be added.

### Skin-Lightening Agents

The compositions of the present invention can also comprise a skin lightening agent. When used, the compositions may comprise from about 0.1% to about 10%, or from about 0.2% to about 5%, or from about 0.5% to about 2%, of a skin lightening agent. Suitable skin lightening agents include those known in the art, including kojic acid, arbutin, ascorbic acid and derivatives thereof, e.g., magnesium ascorbyl phosphate. Further skin lightening agents suitable for use herein also include those described in WO 95/34280 and WO 95/23780.

### Anti-aging additives

The composition can also include an additional anti-aging active such as retinol (Vitamin A) and/or derivative thereof, to enhance repair of photodamage to skin following exposure to ultra-violet light. In addition to retinol itself, examples of derivatives of retinol include: Retinyl acetate, Retinyl butyrate, Retinyl propionate, Retinyl octanoate, Retinyl laurate, Retinyl palmitate, Retinyl oleate, and Retinyl linoleate. The amount of retinol, or a cosmetically acceptable derivative thereof, when present in the composition is from about 0.01 to about 10% or from about 0.1 to about 5% by weight of the composition. The retinols or derivatives may be coupled to the conjugate. Estradiol, estriol, hyaluronic acid, and green tea extract may be incorporated into anti-wrinkle skin cleaning or moisturizing compositions. These formulations may also have ascorbic acid, date palm extract or combinations thereof.

### Particulate Material

Particulate materials, including both organic and inorganic particles, have been included in skin care compositions. See, for example, "Quantification of the Soft-Focus Effect," Cosmetics & Toiletries, Vol. 111, July 1996, pp. 57-61, which discloses that one can physically fill in skin lines with a reflective substance such as TiO₂. Compositions within the scope of the present invention may include particulate materials according to methods and materials described in U.S. Pat. No. 4,892,726, issued to Toshiba Silicone Co. Ltd. Compositions including particulate materials such as polymethylsilsesquioxane powders in makeup and cosmetic compositions are smooth upon application and impart natural colour. An example of such a particle is Tospearl®, available from Toshiba Silicone Co. Ltd. U.S. Pat. No. 5,223,559 describes the use of a variety of particulate fillers of particle size from 0.5 to 50 µm, particularly from 1 to 15 µm, for blurring skin defects.

Particulate materials may have a neat primary particle size from about 2 to about 15 µm, or from about 2 to about 10 µm, or from about 3 to about 7.5 µm. Median particle size can be determined by any suitable method known in the art, such as by using coulter-counter equipment or the ASTM Designation E20-85 "Standard Practice for Particle Size Analysis of Particulate Substances in the Range of 0.2 to 75 Micrometers by Optical Microscopy," ASTM Volume 14.02, 1993.

The particulate materials can be inorganic or organic, such as organosilicone, or organosilicone polymers. Particles may be free-flowing, solid, materials. By "solid" is meant that the particles are not hollow. The void at the centre of hollow particles can have an adverse effect on refractive index and therefore the visual effects of the particles on either skin or the composition.

A representative commercially available example of a particulate material is Tospearl®145 which has a median particle size of about 4.5 [tm. A further representative commercially available example is Orgasol®2002 D NAT COS available from Elf Atochem SA, Paris, France, which is an example of a polyamide organic particulate material. A representative commercially available example of the second particulate material is EA-209® from Kobo which is an ethylene/acrylic acid copolymer having a median particle size of about 10 gm. Other particulate materials that may be used are those made of polymethylsilsesquioxane, referenced above, polyamide, polythene, polyacrylonitrile, polyacrylic acid, polymethacrylic acid, polystyrene, polytetrafluoroethylene (PTFE) and poly(vinylidene chloride). Copolymers derived from monomers of the aforementioned materials can also be used. Inorganic materials include silica and boron nitride.

The compositions of the present invention may comprise one or more particulate materials in a concentration of from about 0.5% to about 25%, or from about 1% to about 20%, or from about 2% to about 12%.

### Other Additives

The composition can also contain adjuncts conventionally used in skin-treating compositions other than those already mentioned, depending on the form of the intended product. It is, for example, possible to include antiseptics, preservatives, and coloring agents, which can improve the stability and consumer appeal of the composition. Examples of other materials include antimicrobials including bactericides and fungicides, acne medication, and wart removers including salicylic acid. Agents known in the art for treatments of conditions such as blisters, insect bites, diaper rash and canker sores may be included; these may be include local anesthetics, emollients, and other known materials.

### pH-Adjusting Agents

The use of one or more pH-adjusting agents, including minor amounts of mineral acids, basic compositions, and organic acids may be used. An exemplary composition includes citric acid, such as is available in an anhydrous salt form of an alkali metal citric acid. The addition of an effective amount of such a pH-adjusting agent is useful in establishing a targeted pH range for compositions according to the invention. The addition of an effective amount of a pH buffering composition so as to maintain the pH of the inventive compositions may also be added. The pH of the final composition is about 4 to about 8, or about 5 to about 7.

Examples of such useful pH buffer compounds and/or pH buffering systems or compositions are alkali metal phosphates, polyphosphates, pyrophosphates, triphosphates, tetraphosphates, silicates, metasilicates, polysilicates, carbonates, hydroxides, and mixtures of the same. Certain salts, such as the alkaline earth phosphates, carbonates, hydroxides, can also function as buffers. It may also be suitable to use as buffers such materials as aluminosilicates (zeolites), borates, aluminates and certain organic materials such as gluconates, succinates, maleates, citrates, and their alkali metal salts. Such buffers keep the pH ranges of the compositions of the present invention within acceptable limits. Others, not particularly elucidated here may also be used. Citric acid, such as is available in an anhydrous salt form of an alkali metal citric acid may be added as it is readily commercially available, and effective. The addition of such a buffering agent is desirable in certain cases wherein long term, i.e., prolonged storage, is to be anticipated for a composition, as well as insuring the safe handling of the aqueous composition.

### Preservatives

Suitable traditional preservatives for compositions of this invention are alkyl esters of parahydroxybenzoic acid. Other preservatives that have more recently come into use include hydantoin derivatives such as 1,3-bis(hydroxymethyl)-5,5-dimthylhydantoin, propionate salts, and a variety of quaternary ammonium compounds such as benzalkonium chloride, quaternium 15 (Dowicil 200), benzethonium chloride, and methylbenzethonium chloride. Cosmetic chemists are familiar with appropriate preservatives and routinely choose them to satisfy the preservative challenge test and to provide product stability. Disodium EDTA, phenoxyethanol, methyl paraben, propyl paraben, imidazolidinyl urea (commercially available as Germall 1157), sodium dehydroacetate or benzyl alcohol may be used with the present invention. The preservatives should be selected having regard for the use of the composition and possible incompatibilities between the preservatives and other ingredients in the emulsion. Preservatives are employed in amounts ranging from about 0% to about 5%, or from about 0.01% to about 2.5%, or from about 0.01% to about 1%, by weight of the composition.

### Water

Where necessary, the compositions further include water sufficient to provide the remaining weight of the composition. Deionized or distilled water may be employed.

### Additional Active Agents that May be Used with the Present Invention

### Ingredients that Alleviate Psoriasis

The present invention may also include active agents that alleviate psoriasis. Such agents should be those that are acceptable for topical application. These agents are known to those of skill in the art. Examples of antipsoriatic agents include, but are not limited to, corticosteroids (e.g., betamethasone dipropionate, betamethasone valerate, clobetasol propionate, diflorasone diacetate, halobetasol propionate, triamcinonide, dexamethasone, fluocinonide, fluocinolone acetonide, halcinonide, triamcinolone acetate, hydrocortisone, hydrocortisone verlerate, hydrocortisone butyrate, aclometasone dipropionate, flurandrenolide, mometasone furoate, and methylprednisolone acetate), methotrexate, cyclosporine, calcipotriene, anthraline, shale oil, elubiol, ketoconazole, coal tar, salicylic acid, zinc pyrithione, selenium sulfide, hydrocortisone, sulfur, menthol, and pramoxine hydrochloride, and combinations thereof. In one embodiment, the compositions of the present invention include an anti-viral agent. Examples of anti-viral agents include, but are not limited to, imiquimod, podofilox, podophyllin, interferon alpha, acyclovir, famcyclovir, valcyclovir, reticulos and cidofovir.

### Antimicrobial Agents

In one embodiment, the compositions of the present invention include an antimicrobial agent. "Antimicrobial agent" as used herein is a compound that kills microorganisms or prevents or inhibits their growth or reproduction. Examples of antimicrobial agents include, but are not limited to ethanol, propanol, betains, benzalkonium chloride, benzethonium chloride, lauric arginayte, sugarquat, methyl benzethonium chloride, cetypyridiunium chloride, 2,4,4',-trichloro-2- hydroxydiphenyl ether (Triclosan), parachlorometa xylenol (PCMX), iodopropynyl butylcarbamate, diazolidinyl urea, chlorhexidene digluconate, chlorhexidene acetate, chlorhexidene isethionate, chlorhexidene hydro-chloride, hexetidine, Quaternium 15, triclocarbon, polyhexamethylene biguanide, cetylpyridium chloride, imidazolidinyl urea, diazolidinyl urea, 3-iodo-2-prommyl-N-butylcarbamate, 2-methyl-4-isothiazolin-3-one, dimethyl dimethyl hydantoin, (5-chloro-2-(2,4-dichlorophenoxy)phenol, monolaurin glyceryl laurate, camellia sinensis, candida bombi-cola/glucose/methyl rapeseedate ferment, hydrogen peroxide, phenol, poloxamer 188, PVP-iodine, thiourea, natural antimicrobial agents, such as cinnamon oil, cinnamaldehyde, lemongrass oil, clove oil, saw palmetto extract, thyme oil white, thyme oil red, thymol, tea free oil, pinus pinaster bark extract, rosemary leaf extract, grape seed extract, and betel oil, silver containing compounds, such as silver nitrate, silver lactate, silver citrate, and silver zeolite, antimicrobial fatty acid ester of a polyhydric alcohol, a fatty ether of a polyhydric alcohol and alkoxylated derivatives thereof, and combinations thereof.

Bactericidal agents used in the present invention may include enzymatic enzymes. These may include any member from the class of oxido-reductases, EC 1 that generate active oxygen; Monosasccharide oxidases, Peroxidases, Lactoperoxidases, Salivary peroxidases, Myeloperoxidases, Phenol oxidase, Cytochrome oxidase, Dioxygenases, Monooxygenases. The enzymes also include bacterial cell lytic enzymes, e.g., Lysozyme, Lactoferrin. Other agents include antimicrobials e.g., chlorhexidine, amine fluoride compounds, fluoride ions, hypochlorite, quaterinary ammonium compounds e.g. cetylpyridinium chloride, hydrogen peroxide, monochloramine, providone iodine, any recognized sanitizing agent or oxidative agent and biocides.

Compositions of the present invention may also include antibiotics including, but not limited to the following classes and members within a class: Aminoglycosides: Gentamicin, Tobramycin, Netilmicin, Amikacin, Kanamycin, Streptomycin, Neomycin; Quinolones/Fluoroquinolones: Nalidixic Acid, Cinoxacin, Norfloxacin, Ciprofloxacin, Perfloxacin, Ofloxacin, Enoxacin, Fleroxacin, Levofloxacin; Antipseudomonal: Carbenicillin, Carbenicillin Indanyl, Ticarcillin, Azlocillin, Mezlocillin, Piperacillin; Cephalosporins: Cephalothin, Cephaprin, Cephalexin, Cephradine, Cefadroxil, Cefazolin, Cefamandole, Cefoxitin, Cefaclor, Cefuroxime, Cefotetan, Ceforanide, Cefuroxine Axetil, Cefonicid, Cefotaxime, Moxalactam, Ceftizoxime, Ceftriaxone, Cefoperazone, Cftazidime, Cephaloridine, Cefsulodin; Other beta-Lactam Antibiotics: Imipenem, Aztreonam; beta-Lactamase Inhibitors: Clavulanic Acid, Augmentin, Sulbactam; Sulfonamides: Sulfanilamide, Sulfamethoxazole, Sulfacetamide, Sulfadiazine, Sulfisoxazole, Sulfacytine, Sulfadoxine, Mafenide, p-Aminobenzoic Acid, Trimethoprim-Sulfamethoxazole; Urinary Tract Antiseptics: Methenamine, Nitrofurantoin, Phenazopyridine and other napthpyridines; Penicillins: Penicillin G and Penicillin V; Penicillinase Resistant: Methicillin, Nafcillin, Oxacillin, Cloxacillin, Dicloxacillin; Penicillins for Gram-Negative/Amino Penicillins: Ampicillin (Polymycin), Amoxicillin, Cyclacillin, Bacampicillin; Tetracyclines: Tetracycline, Chlortetracycline, Demeclocycline, Methacycline, Doxycycline, Minocycline; Other Antibiotics: Chloramphenicol (Chlonnycetin), Erythromycin, Lincomycin, Clindamycin, Spectinomycin, Polymyxin B (Colistin), Vancomycin, Bacitracin; Tuberculosis Drugs: Isoniazid, Rifampin, Ethambutol, Pyrazinamide, Ethinoamide, Aminosalicylic Acid, Cycloserine; Anti-Fungal Agents: Amphotericin B, Cyclosporine, Flucytosine; Imidazoles and Triazoles: Ketoconazole, Miconazaole, Itraconazole, Fluconazole, Griseofulvin; Topical Anti Fungal Agents: Clotrimazole, Econazole, Miconazole, Terconazole, Butoconazole, Oxiconazole, Sulconazole, Ciclopirox Olamine, Haloprogin, Tolnaftate, Naftifine, Polyene, Amphotericin B, Natamycin.

In one embodiment, the amount of antimicrobial agent in the compositions is from about 0.001% to about 10%, such as from about 0.01% to about 5%, such as from about 0.05% to about 2% by weight, based on the total weight of the composition.

In one embodiment the antimicrobial agent is an anti-fungal agent such as an azole. Examples include, but are not limited to, miconazole, ketoconazole, econazole, itraconazole, sertaconazole, fluconazole, voriconazole, clioquinol, bifoconazole, terconazole, butoconazole, tioconazole, oxiconazole, sulconazole, saperconazole, clotrimazole, unde-cylenic acid, haloprogin, butenafine, tolnaftate, nystatin, ciclopirox olamine, terbinafine, amorolfine, naftifine, elubiol, griseofulvin, their cosmetically acceptable salts, and combinations thereof. In one embodiment the antimicrobial agent is an antibiotic or an antiseptic. Examples include, but are not limited to, mupirocin, neomycin sulfate bacitracin, polymyxin B, 1-ofloxacin, tetracyclines such as chlortetracycline hydrochloride, oxytetracycline - 10 hydrochloride and tetracycline hydrochoride, clindamycin phosphate, gentamicin sulfate, metronidazole, hexylresorcinol, methylbenzethonium chloride, phenol, quaternary ammonium compounds, tea tree oil, and combinations thereof.

### Anti-Inflammatory Agents

In another embodiment, the compositions of the present invention may include an anti-inflammatory agent. A safe and effective amount of an anti-inflammatory agent may be added to the compositions of the subject invention, from about 0.1% to about 5%, or from about 0.1% to about 2%, of the composition. The anti-inflammatory agent enhances the skin appearance benefits of the present invention, e.g., such agents contribute to a more uniform and acceptable skin tone or colour. The exact amount of anti-inflammatory agent to be used in the compositions will depend on the particular anti-inflammatory agent utilized since such agents vary widely in potency.

Anti-inflammatory agents useful herein include steroids such as hydrocortisone; panthenol and ether and ester derivatives thereof e.g. panthenol ethyl ether, panthenyl triacetate; pantothenic acid and salt and ester derivatives thereof, such as calcium pantothenate; aloe vera, bisabolol, allantoin and compounds of the liquorice (the plant genus/species Glycyrrhiza glabra) family, including glycyrrhetic acid, glycyrrhizic acid, and derivatives thereof e.g. salts such as ammonium glycyrrhizinate and esters such as stearyl glycyrrhetinate. Suitable levels may be from about 0.1 to about 5%, or from about 0.5 to about 3%. Additional anti-inflammatory agents include, but are not limited to, corticosteroids such as hydrocortisone, hydroxyltriamcinolone alphamethyl dexamethasone, dexamethasone-phosphate, beclomethasone dipropionate, clobe-tasol valerate, desonide, desoxymethasone, desoxycorticosterone acetate, dexamethasone, dichlorisone, diflorasone diacetate, diflucortolone valerate, fluadrenolone, fluclarolone acetonide, fludrocortisone, flumethasone pivalate, fluosi-nolone acetonide, fluocinonide, flucortine butylester, fluocortolone, fluprednidene (fluprednylidene)acetate, flurandre-nolone, halcinonide, hydrocortisone acetate, hydrocortisone butyrate, methylprednisolone, triamcinolone acetonide, cortison, cortodoxone, flucetonide, fludrocortisone, difluorosone diacetate, fluradrenalone acetonide, medrysone, am-ciafel, amcinafide, betamethasone, chlorprednisone, chlorprednisone acetate, clocortelone, clescinolone, dichlorisone, difluprednate, flucloronide, flunisolide, fluoromethalone, fluperolone, fluprednisolone, hydrocortisone valerate, hydrocortisone cyclopentylproprionate, hydrocortamate, meprednisone, paramethasone, prednisolone, prednisone, beclomethasone dipropionate, betamethasone dipropionate, and triamcinolone, and combinations thereof.

Other active agents that may be incorporated into embodiments of the present invention include, but are not limited to, wound healing enhancing agents such as calcium alginate, collagen, recombinant human platelet-derived growth factor (PDGF) and other growth factors, ketanserin, iloprost, prostaglandin El and hyaluronic acid; scar reducing agents such as mannose-6-phosphate; analgesie agents; debriding agents such as papain, and enzymatic debriding agents; and anesthetics such as lidocaine and benzocaine. In one embodiment, the composition comprises one or more of menthol, camphor, an antihistamine, or a local anesthetic such as tetracaine, lidocaine, prilocaine, benzocaine, bupivacaine, mepivacaine, dibucaine, etidocaine, butacaine, cyclomethycaine, hexylcaine, proparacaine, and lopivacaine, capsaicin, or oatmeal.

In one embodiment, the amount of anti-inflammatory agent, anti-viral agent, anti-psoriatic agent and/or other active agent in the compositions is from about 0.001% to about 10%, such as from about 0.01% to about 5% such as from about 0.05% to about 2% by weight, based on the total weight of the composition.

### Preparation of Compositions

The compositions of the present invention are generally prepared by conventional methods such as are known in the art of making topical compositions. Such methods typically involve mixing of the ingredients in one or more steps to a relatively uniform state, with or without heating, cooling, application of vacuum, and the like.

### Methods for Regulating Skin Condition

The compositions of the present invention are useful for regulating mammalian skin condition, for example human skin, including the face, scalp, and hands or other non-facial parts of the body, including regulating visible and/or tactile discontinuities in skin, e.g., visible and/or tactile discontinuities in skin texture, such as discontinuities associated with skin ageing. Regulating skin condition involves topically applying to the skin a safe and effective amount of a composition of the present invention. The amount of the composition which is applied, the frequency of application and the period of use wilt vary widely depending upon the active levels of a given composition and the level of regulation desired, e.g., in light of the level of skin ageing present in the subject and the rate of further skin ageing.

A wide range of quantities of the compositions of the present invention can be employed to provide a skin appearance and/or feel benefit. Quantities of the present compositions which are typically applied per application are, in mg composition/cm² skin, from about 0.1 mg/cm² to about 10 mg/cm². A particularly useful application amount is about 2 mg/cm². Typically applications would be on the order of about once per day, however application rates can vary from about once per week up to about three times per day or more.

The compositions of this invention provide a visible improvement in skin condition essentially immediately following application of the composition to the skin. Such immediate improvement involves coverage or masking of skin imperfections such as textural discontinuities (including those associated with skin ageing,, such as enlarged pores), and/or providing a more even skin tone or color.

The compositions of the invention also provide visible improvements in skin condition following chronic topical application of the composition. "Chronic topical application" and the like involves continued topical application of the composition over an extended period during the subject's lifetime, for a period of at least about one week, or for a period of at least about one month, or for at least about three months, or for at least about six months, or for at least about one year. Chronic regulation of skin condition involves improvement of skin condition following multiple topical applications of the composition to the skin. Typically applications would be on the order of about once per day over such extended periods, however application rates can vary from about once per week up to about three times per day or more.

Regulating skin condition may be practiced by applying a composition in the form of a skin lotion, cream, cosmetic, or the like which is intended to be left on the skin for an extended period for some aesthetic, prophylactic, therapeutic or other benefit (i.e., a "leave-on" composition). As used herein, "leave-on" compositions exclude rinse-off skin cleansing products. After applying the composition to the skin, the leave-on composition may be left on the skin for a period of at least about 15 minutes, or at least about 30 minutes, or at least about 1 hour, or for at least several hours, e.g., up to about 12 hours.

### EXAMPLES

The compositions of the present invention are generally prepared by conventional methods such as are known in the art of making topical compositions. Such methods typically involve mixing of the ingredients in one or more steps to a relatively uniform state, with or without heating, cooling, application of vacuum and the like.

The following examples further describe and demonstrate embodiments within the scope of the present invention. The examples are given solely for the purpose of illustration and are not to be construed as limitations of the present invention.

### Example 1: Oil-in-water Emulsions

The following emulsion is prepared using conventional formulating techniques.

| | 1 %w/w | 2 %w/w | 3 %w/w |
|---|---|---|---|
| Meta Silicate | 0.21 | 0.31 | 0.42 |
| Sodium Carbonate | 0.16 | 0.22 | 0.32 |
| Sodium Gluconate | 0.06 | 0.09 | 0.13 |
| Potassium Aluminium Sulfate | 0.06 | 0.09 | 0.13 |
| Tospearl®145 | 6.0 | 9.0 | 3.0 |
| Polyacrylamide (thickening agent) | 2.0 | 2.0 | 1.0 |
| Xanthan Gum (thickening agent) | | 0.6 | 0.3 |
| Glycerine (plasticizing agent/humectant) | 7.0 | 5.0 | 3.0 |
| Urea (antimicrobial) | 0.0 | 0.0 | 2.0 |
| Panthenol (hair conditioning agent) | 1.0 | 0.0 | 0.0 |
| Salicylic acid (anti- acne agent) | 0.0 | 1.5 | 0.0 |
| Allantoin (anti-inflammatory agent) | 0.2 | 0.1 | 0.0 |
| Aloe Vera gel (anti-inflammatory) | 0.0 | 0.0 | 0.05 |
| Tocopheryl acetate | 0.0 | 0.0 | 0.05 |
| Cetyl Alcohol (emollient) | 2.0 | 1.0 | 1.25 |
| Stearyl alcohol (emollient) | 2.0 | 1.0 | 1.25 |
| Cyclomethicone (emollient) and dimethiconol | 0.75 | 0.5 | 0.50 |
| Steareth-21 (surfactant) | 0.6 | 0.4 | 0.3 |
| Steareth-2 (surfactant) | 0.1 | 0.08 | 0.03 |
| Sorbitan stearate & sucrose cocoate (surfactants) | 1.5 | 0 | 0 |
| PPG-15 stearyl ether (emollient) | 3.0 | 5.0 | 4.0 |
| Sucrose polycottonseedate (emollient) | 2.0 | 3.0 | 0 |
| Dimethicone (silicone conditioning agent) | 0.5 | 0 | 0 |
| Disodium EDTA | 0.02 | 0.01 | 0.02 |
| Deionized water | To 100% | To 100% | To 100% |

### Example 2

### Moisturizing Lotion

| | |
|---|---|
| Meta Silicate | 0.31 |
| Sodium Carbonate | 0.22 |
| Sodium Gluconate | 0.09 |
| Potassium Aluminium Sulfate | 0.09 |
| Cyclomethicone (emollient) | 17.35 |
| Propylparaben (preservative) | 0.20 |
| Ethylene/Acrylic Acid Copolymer microspheres (e.g., Flobeads EA 209 supplied by Kobo Products) | 10.0 |
| Glycerin (plasticizing agent/humectant) | 25.00 |
| Niacinamide (Vitamin B3) | 3.00 |
| Methylparaben (preservative) | 0.12 |
| Water | to 100 % |

In a suitable vessel, the meta silicate, sodium carbonate, sodium gluconate, potassium A1 sulfate are mixed with the niacinamide and water until homogeneous. To this mixture is added the glycerin, ethylene/acrylic acid copolymenr microspheres and methylparaben with mixing until homogenous. The Mixture is then combined with the cyclomethicone and mixed using conventional mixing methods until homogenous. The mixture is then poured into suitable containers. The moisturizing cosmetic lotion is applied to the face and/or body to provide softening, moisturization and conditioning.

### Example 3 (not according to the invention)

### Liquid Formulation

| | |
|---|---|
| Meta Silicate | 0.31 |
| Sodium Carbonate | 0.22 |
| Sodium Gluconate | 0.09 |
| Sea salt | 0.09 |
| Cyclomethicone | 11.62 |
| Dimethicone copolyol emulsifier | 0.7 |
| Isononyl isononanoate (emollient) | 5.00 |
| n-Propyl-4-hydroxybenzoic acid (anti-fungal/preservative) | 0.20 |
| Fragrance | 0.03 |
| Titanium dioxide (pigment/sunscreen) | 17.8 |
| Yellow iron oxide (pigment) | 1.70 |
| Red iron oxide (pigment) | 0.19 |
| Black iron oxide (pigment) | 0.11 |
| Methylparahydroxybenzoate (preservative) | 0.12 |
| Glycerin | 10.0 |
| 2-Amino-2-methyl-1-propanol | 0.10 |
| Sucrose oleate ester (emollient) | 0.60 |
| Water | to 100% |

## Claims

1. A composition for use in treating skin damage, comprising:
(a) one or more metasilicate selected from the group consisting of sodium or potassium metasilicate, sodium or potassium orthosilicate and mixtures thereof;
(b) one or more carbonate selected from the group consisting of sodium carbonate, sodium sesquicarbonate, sodium bicarbonate and mixtures thereof;
(c) one or more gluconate;
(d) one or more sulfate including potassium aluminium sulfate, wherein the one or more sulfate is further selected from the group consisting of sodium sulfate, potassium sulfate, lithium sulfate, ammonium sulfate, magnesium sulfate, strontium sulfate, aluminium sulfate, and mixtures thereof; and
(e) optionally one or more salt selected from the group consisting of sea salt and sodium chloride.

2. The composition of claim 1, wherein the skin damage is selected from the group consisting of eczema, atopic dermatitis, contact dermatitis, seborrhea, xerosis, rosacea, thermal or radiation burns, psoriasis and inflammation.

3. The composition of claim 1, wherein the composition further comprises an emulsifying agent, a surfactant, a thickening agent, or a mixture thereof.

4. The composition of claim 1, wherein the one or more gluconate is selected from the group consisting of ammonium gluconate, lithium gluconate, sodium gluconate, sodium starch gluconate, potassium gluconate, ammonium acid gluconate, sodium acid gluconate, lithium acid gluconate, potassium acid gluconate, ammonium D-gluconate, lithium D-gluconate, sodium D-gluconate, potassium D-gluconate, gluconic acid, gluconic D-acid, gluconic L-acid, ammonium L-gluconate, lithium L-gluconate, sodium L-gluconate, potassium L-gluconate, magnesium gluconate, magnesium acid gluconate, magnesium D-gluconate, magnesium L-gluconate, calcium gluconate, calcium acid gluconate, calcium D-gluconate, calcium L-gluconate and mixtures thereof.

5. The composition of claim 1, wherein the composition comprises
a) from about 0.11% wt. to about 0.84% wt., preferably about 0.21% wt. to about 0.42% wt., of a metasilicate,
b) from about 0.08% wt. to about 0.64% wt., preferably from about 0.16% wt. to about 0.32% wt., of a carbonate,
c) from about 0.03% wt. to about 0.26% wt., preferably from about 0.06% wt. to about 0.13% wt., of a gluconate, and
d) from about 0.03% wt. to about 0.26% wt., preferably from about 0.06% wt. to about 0.13% wt. of a sulfate.

6. The composition of claim 1, wherein the carbonate is sodium carbonate, the gluconate is sodium gluconate, and the sulfate is potassium aluminium sulfate.

7. The composition of claim 1, wherein the composition further comprises a physiologically acceptable carrier, preferably selected from liposomes, solutions, creams, emollients, ointments, gels, solid formulations and liquid formulations, or diluents.

8. The composition of claim 1, wherein the composition is in the form of a liquid, cream, oil, gel, fluid cream, lotion, emulsion or micro-emulsion.

9. The composition of claim 1, wherein the composition further comprises an active drug substance, preferably selected from a corticosteroid, metronidazole, sulfacetamide, sulfur, or azelaic acid or a combination thereof, or an active cosmetic substance selected from the group consisting of desquamatory actives, anti-acne actives, retinoids, peptides, hydroxy acids, anti-oxidants, radical scavengers, chelators, anti-inflammatory agents, topical anesthetics, antimicrobial actives, skin soothing agents, skin healing agents, antifungal actives, thickening agents, and mixtures thereof.

10. The composition of claim 9, wherein the active cosmetic substance is retinol, a retinol derivative, or a mixture thereof, allantoin, retinol propionate, tocopherol, tocopherol derivatives, tocopherol esters, peptides, peptide derivatives, niacinamide, phytosterols, isoflavones, panthenol, panthenol derivatives, salicylic acid, bisabolol or farnesol.

11. The composition of claim 8, wherein the composition further comprises an effective amount of an active agent selected from the group consisting of psoriasis-alleviating agents, anti-microbial agents, anti-inflammatory agents or a combination thereof.

12. The composition of claim 8, wherein the concentration is in an amount effective to treat skin damage.

13. The composition of claim 8, wherein the composition comprises a) from about 0.04% wt. to about 1.6% wt. of a metasilicate, b) from about 0.03% wt. to about 1.2% wt. of a carbonate, c) from about 0.01 % wt. to about 0.04% wt. of a gluconate, and d) from about 0.01% wt. to about 0.04% wt. of a sulfate.

14. Use of the composition of any of claims 1-13 for the preparation of a medicament for the treatment of skin damage preferably selected from the group consisting of eczema, atopic dermatitis, contact dermatitis, seborrhea, xerosis, rosacea, thermal or radiation burns, psoriasis and inflammation by topically applying to the skin a composition containing an effective amount of the composition.

## Patentansprüche

1. Zusammensetzung zur Verwendung bei der Behandlung eines Hautschadens, umfassend:
(a) ein oder mehrere Metasilikate ausgewählt aus der Gruppe bestehend aus Natrium- oder Kaliummetasilikat, Natrium- oder Kaliumorthosilikat und Gemischen davon;
(b) ein oder mehrere Carbonate ausgewählt aus der Gruppe bestehend aus Natriumcarbonat, Natriumsesquicarbonat, Natriumbicarbonat und Gemischen davon;
(c) ein oder mehrere Gluconate;
(d) ein oder mehrere Sulfate umfassend Kaliumaluminiumsulfat, wobei das eine oder die mehreren Sulfate ferner aus der Gruppe bestehend aus Natriumsulfat, Kaliumsulfat, Litiumsulfat, Ammoniumsulfat, Magnesiumsulfat, Strontiumsulfat, Aluminiumsulfat und Gemischen davon ausgewählt sind; und
(e) optional ein oder mehrere Salze ausgewählt aus der Gruppe bestehend aus Meersalz und Natriumchlorid.

2. Zusammensetzung nach Anspruch 1, wobei der Hautschaden ausgewählt ist aus der Gruppe bestehend aus Ekzem, atopischer Dermatitis, Kontaktdermatitis, Seborrhoe, Xerose, Rosacea, thermischen oder Strahlenverbrennungen, Psoriasis und Entzündung.

3. Zusammensetzung nach Anspruch 1, wobei die Zusammensetzung ferner ein Emulgiermittel, ein Tensid, ein Verdickungsmittel oder ein Gemisch davon umfasst.

4. Zusammensetzung nach Anspruch 1, wobei das eine oder die mehreren Gluconate ausgewählt sind aus der Gruppe bestehend aus Ammoniumgluconat, Litiumgluconat, Natriumgluconat, Natriumstärkegluconat, Kaliumgluconat, Ammoniumsäuregluconat, Natriumsäuregluconat, Lithiumsäuregluconat, Kaliumsäuregluconat, Ammonium-D-Gluconat, Lithium-D-Gluconat, Natrium-D-Gluconat, Kalium-D-Gluconat, Gluconsäure, Glucon-D-Säure, Glucon-L-Säure, Ammonium-L-Gluconat, Lithium-L-Gluconat, Natrium-L-Gluconat, Kalium-L-Gluconat, Magnesiumgluconat, Magnesiumsäuregluconat, Magnesium-D-Gluconat, Magnesium-L-Gluconat, Calciumgluconat, Calciumsäuregluconat, Calcium-D-Gluconat, Calcium-L-Gluconat und Gemischen davon.

5. Zusammensetzung nach Anspruch 1, wobei die Zusammensetzung umfasst:
(a) von etwa 0,11 Gew-% bis etwa 0,84 Gew-%, bevorzugt etwa 0,21 Gew-% bis etwa 0,42 Gew-% eines Metasilikats,
(b) von etwa 0,08 Gew-% bis etwa 0,64 Gew-%, bevorzugt etwa 0,16 Gew-% bis etwa 0,32 Gew-% eines Carbonats,
(c) von etwa 0,03 Gew-% bis etwa 0,26 Gew-%, bevorzugt von etwa 0,06 Gew-% bis etwa 0,13 Gew-% eines Gluconats, und
(d) von etwa 0,03 Gew-% bis etwa 0,26 Gew-%, bevorzugt von etwa 0,06 Gew-% bis etwa 0,13 Gew-% eines Sulfats.

6. Zusammensetzung nach Anspruch 1, wobei das Carbonat Natriumcarbonat ist, das Gluconat Natriumgluconat ist, und das Sulfat Kaliumaluminiumsulfat ist.

7. Zusammensetzung nach Anspruch 1, wobei die Zusammensetzung ferner einen physiologisch verträglichen Träger, bevorzugt ausgewählt aus Liposomen, Lösungen, Cremes, Weichmachern, Salben, Gelen, festen Formulierungen und flüssigen Formulierungen oder Verdünnungsmitteln.

8. Zusammensetzung nach Anspruch 1, wobei die Zusammensetzung in Form einer Flüssigkeit, Creme, Öl, Gel, flüssigen Creme, Lotion, Emulsion oder Mikroemulsion vorliegt.

9. Zusammensetzung nach Anspruch 1, wobei die Zusammensetzung ferner einen aktiven Wirkstoff umfasst, bevorzugt ausgewählt aus einem Kortikosteriod, Metrondiazol, Sulfacetamid, Schwefel oder Azelainsäure oder eine Kombination davon, oder eine aktive kosmetische Substanz ausgewählt aus der Gruppe bestehend aus Desquamationswirkstoffen, Anti-Akne Wirkstoffen, Retinoiden, Peptiden, Hydroxysäuren, Antioxidantien, Radikalfängern, Chelatoren, entzündungshemmenden Mitteln, topischen Anästhetika, Antimikrobiellen Mitteln, Hautberuhigungsmitteln, Hautheilmittel, Antimykotika, Verdickungsmittel und Gemischen davon.

10. Zusammensetzung nach Anspruch 9, wobei die aktive kosmetische Substanz Retinol, ein Retinolderivat oder eine Mischung davon, Allantoin, Retinolpropionate, Tocopherol, Tocopherolderivate, Tocopherolester, Peptide, Peptidderivate, Niacinamid, Phytosterole, Isoflavone, Panthenol, Phanthenolderivate, Salizylsäure, Bisabolol oder Farnesol ist.

11. Zusammensetzung nach Anspruch 8, wobei die Zusammensetzung ferner eine wirksame Menge eines aktiven Wirkstoffs ausgewählt aus der Gruppe bestehend aus Psoriasis-lindernden Mitteln, antimikrobiellen Mitteln, entzündungshemmenden Mitteln oder einer Kombination davon umfasst.

12. Zusammensetzung nach Anspruch 8, wobei die Konzentration in einer Menge wirksam, um einen Hautschaden zu behandeln, vorliegt.

13. Zusammensetzung nach Anspruch 8, wobei die Zusammensetzung umfasst a) von etwa 0,04 Gew-% bis etwa 1,6 Gew-% eines Metasilikats, b) von etwa 0,03 Gew-% bis etwa 1,2 Gew-% eines Carbonats, c) von etwa 0,01 Gew-% bis etwa 0,04 Gew-% eines Gluconats, und d) von etwa 0,01 Gew-% bis etwa 0,04 Gew-% eines Sulfonats.

14. Verwendung der Zusammensetzung nach einem der Ansprüche 1 bis 13 zur Herstellung eines Medikaments zur Behandlung eines Hautschadens, bevorzugt ausgewählt aus der Gruppe bestehend aus Ekzem, Atopische Dermatitis, Kontaktdermatitis, Seborrhoe, Xerose, Rosacea, thermischen oder Strahlenverbrennungen und Psoriasis und Entzündung, durch topisches Auftragen einer Zusammensetzung, welche eine wirksame Menge der Zusammensetzung enthält, auf die Haut.

## Revendications

1. Composition destinée à être utilisée dans le traitement des lésions cutanées, comprenant :
(a) un ou plusieurs métasilicates choisis dans le groupe constitué de métasilicate de sodium ou de potassium, orthosilicate de sodium ou de potassium et leurs mélanges;
(b) un ou plusieurs carbonates choisis dans le groupe constitué de carbonate de sodium, sesquicarbonate de sodium, bicarbonate de sodium et leurs mélanges;
(c) un ou plusieurs gluconates;
(d) un ou plusieurs sulfates, y compris du sulfate de potassium et d'aluminium, dans lequel le ou les sulfates sont en outre choisis dans le groupe constitué de sulfate de sodium, sulfate de potassium, sulfate de lithium, sulfate d'ammonium, sulfate de magnésium, sulfate de strontium, sulfate d'aluminium et leurs mélanges; et
(e) éventuellement un ou plusieurs sels choisis dans le groupe constitué de sel marin et chlorure de sodium.

2. Composition selon la revendication 1, dans laquelle les lésions cutanées sont choisies dans le groupe constitué d'eczéma, dermatite atopique, dermatite de contact, séborrhée, xérose, rosacée, brûlures thermiques ou par rayonnement, psoriasis et inflammation.

3. Composition selon la revendication 1, dans laquelle la composition comprend en outre un agent émulsifiant, un agent tensioactif, un agent épaississant ou un mélange de ceux-ci.

4. Composition selon la revendication 1, dans laquelle le ou les gluconates sont choisis dans le groupe constitué de gluconate d'ammonium, gluconate de lithium, gluconate de sodium, gluconate d'amidon de sodium, gluconate de potassium, gluconate acide d'ammonium, gluconate acide de sodium, gluconate acide de lithium, gluconate acide de potassium, D-gluconate d'ammonium, D-gluconate de lithium, D-gluconate de sodium, D-gluconate de potassium, acide gluconique, D-acide gluconique, L-acide gluconique, L-gluconate d'ammonium, L-gluconate de lithium, L-gluconate de sodium, L-gluconate de potassium, gluconate de magnésium, gluconate acide de magnésium, D-gluconate de magnésium, L-gluconate de magnésium, gluconate de calcium, gluconate acide de calcium, D-gluconate de calcium, L-gluconate de calcium et leurs mélanges.

5. Composition selon la revendication 1, dans laquelle la composition comprend
a) d'environ 0,11 % en poids à environ 0,84 % en poids, de préférence d'environ 0,21 % en poids à environ 0,42 % en poids, d'un métasilicate,
b) d'environ 0,08 % en poids à environ 0,64 % en poids, de préférence d'environ 0,16 % en poids à environ 0,32 % en poids, d'un carbonate,
c) d'environ 0,03 % en poids à environ 0,26 % en poids, de préférence d'environ 0,06 % en poids à environ 0,13 % en poids, d'un gluconate, et
d) d'environ 0,03 % en poids à environ 0,26 % en poids, de préférence d'environ 0,06 % en poids à environ 0,13 % en poids d'un sulfate.

6. Composition selon la revendication 1, dans laquelle le carbonate est du carbonate de sodium, le gluconate est du gluconate de sodium et le sulfate est du sulfate d'aluminium de potassium.

7. Composition selon la revendication 1, dans laquelle la composition comprend en outre un support physiologiquement acceptable, de préférence choisi parmi les liposomes, les solutions, les crèmes, les émollients, les pommades, les gels, les formulations solides et les formulations liquides, ou les diluants.

8. Composition selon la revendication 1, dans laquelle la composition se présente sous la forme d'un liquide, d'une crème, d'une huile, d'un gel, d'une crème fluide, d'une lotion, d'une émulsion ou d'une micro-émulsion.

9. Composition selon la revendication 1, dans laquelle la composition comprend en outre une substance médicamenteuse active, de préférence choisie parmi un corticostéroïde, métronidazole, sulfacétamide, soufre ou acide azélaïque ou une combinaison de ceux-ci, ou une substance cosmétique active choisie dans le groupe constitué d'actifs desquamants, actifs anti-acnéiques, rétinoïdes, peptides, hydroxyacides, antioxydants, capteurs de radicaux, chélateurs, agents anti-inflammatoires, anesthésiques topiques, actifs antimicrobiens, agents apaisants pour la peau, agents de cicatrisation de la peau, actifs antifongiques, agents épaississants, et leurs mélanges.

10. Composition selon la revendication 9, dans laquelle la substance cosmétique active est le rétinol, un dérivé du rétinol ou un mélange de ceux-ci, l'allantoine, le propionate de rétinol, le tocophérol, les dérivés du tocophérol, les esters de tocophérol, les peptides, les dérivés de peptides, la niacinamide, les phytostérols, les isoflavones, le panthénol, les dérivés du panthénol, l'acide salicylique, le bisabolol ou le farnésol.

11. Composition selon la revendication 8, dans laquelle la composition comprend en outre une quantité efficace d'un agent actif choisi dans le groupe constitué d'agents soulageant le psoriasis, agents antimicrobiens, agents anti-inflammatoires ou une combinaison de ceux-ci.

12. Composition selon la revendication 8, dans laquelle la concentration est en une quantité efficace pour traiter les dommages cutanés.

13. Composition selon la revendication 8, dans laquelle la composition comprend a) d'environ 0,04 % en poids à environ 1,6 % en poids d'un métasilicate, b) d'environ 0,03 % en poids à environ 1,2 % en poids d'un carbonate, c) d'environ 0,01 % en poids à environ 0,04 % en poids d'un gluconate, et d) d'environ 0,01 % en poids à environ 0,04 % en poids d'un sulfate.

14. Utilisation de la composition selon l'une quelconque des revendications 1 à 13 pour la préparation d'un médicament pour le traitement des lésions cutanées, de préférence choisi dans le groupe constitué d'eczéma, dermatite atopique, dermatite de contact, séborrhée, xérose, rosacée, brûlures thermiques ou par irradiation, psoriasis et inflammation, par application topique sur la peau d'une composition contenant une quantité efficace de la composition.
